# EUROPEAN PATENT APPLICATION

(11) **EP 2 878 309 A1**
(43) Date of publication of application: **03.06.2015**
(21) Application number: 13823500.7
(22) Date of filing: 12.07.2013
(51) Int. Cl.: A61K 45/00, A61K 31/4439, A61K 31/47, A61K 31/7048, A61P 17/00, A61P 37/08, C12Q 1/68, G01N 33/15, G01N 33/50, C07K 14/705, C12N 15/09

(54) **FILAGGRIN PRODUCTION PROMOTER, THERAPEUTIC AGENT FOR DISEASES ASSOCIATED WITH REDUCTION IN PRODUCTION OF FILAGGRIN, AND METHOD FOR SCREENING FOR SAID THERAPEUTIC AGENT**

(30) Priority: 26.07.2012 JP 2012165680
(71) Applicant: Kyoto University, Kyoto-shi, Kyoto 606-8501 (JP); Astellas Pharma Inc., Chuo-ku Tokyo 103-8411 (JP)
(72) Inventor: KABASHIMA, Kenji, Kyoto-shi Kyoto 606-8501 (JP); OTSUKA, Atsushi, Kyoto-shi Kyoto 606-8501 (JP); EGAWA, Gyohei, Kyoto-shi Kyoto 606-8501 (JP); DOI, Hiromi, Kyoto-shi Kyoto 606-8501 (JP); MAEKAWA, Akiko, Kyoto-shi Kyoto 606-8501 (JP); NARUMIYA, Shuh, Kyoto-shi Kyoto 606-8501 (JP)
(74) Representative: Brantsandpatents bvba
(86) International application number: PCT/JP2013/069163
(87) International publication number: WO 2014/017319

(57) **Abstract**

The present invention provides a highly effective substance having a filaggrin production-promoting action. The present invention also provides a therapeutic agent useful for treating a skin disease, in particular, atopic dermatitis associated with reduced filaggrin production. As a result of intensive studies on a filaggrin production promoter, it has been found that A) an N-quinolylbenzamide derivative (Compound 1), B) ivermectin (Compound 2), or C) a phenylalaninamide derivative (Compound 3) promotes production of filaggrin in skin and satisfactorily improves a water retention ability and barrier function of the stratum corneum. The present invention relates to a filaggrin production promoter containing a certain N-quinolylbenzamide derivative or a pharmaceutically acceptable salt thereof as an active ingredient.

SEQUENCE LISTING

<110>  Kyoto University
       Astellas Pharma Inc.

<120>  Filaggrin production promoter, a therapeutic agent for diseases
       associated with impaired production of filaggrin, and a method
       for screening of the therapeutic agent thereof

<130>  GP13-1007PCT

<150>  2012-165680
<151>  2012-07-26

<160>  28

<170>  PatentIn version 3.5

<210>  1
<211>  462
<212>  PRT
<213>  Homo sapiens

<400>  1

Met Ser Ile Met Met Leu Pro Met Glu Gln Lys Ile Gln Trp Val Pro
1               5                   10                  15

Thr Ser Leu Gln Asp Ile Thr Ala Val Leu Gly Thr Glu Ala Tyr Thr
            20                  25                  30

Glu Glu Asp Lys Ser Met Val Ser His Ala Gln Lys Ser Gln His Ser
        35                  40                  45

Cys Leu Ser His Ser Arg Trp Leu Arg Ser Pro Gln Val Thr Gly Gly
    50                  55                  60

Ser Trp Asp Leu Arg Ile Arg Pro Ser Lys Asp Ser Ser Ser Phe Arg
65                  70                  75                  80

Gln Ala Gln Cys Leu Arg Lys Asp Pro Gly Ala Asn Asn His Leu Glu
                85                  90                  95

Ser Gln Gly Val Arg Gly Thr Ala Gly Asp Ala Asp Arg Glu Leu Arg
            100                 105                 110

Gly Pro Ser Glu Lys Ala Thr Ala Gly Gln Pro Arg Val Thr Leu Leu
        115                 120                 125

Pro Thr Pro His Val Ser Gly Leu Ser Gln Glu Phe Glu Ser His Trp
    130                 135                 140

Pro Glu Ile Ala Glu Arg Ser Pro Cys Val Ala Gly Val Ile Pro Val
145                 150                 155                 160

Ile Tyr Tyr Ser Val Leu Leu Gly Leu Gly Leu Pro Val Ser Leu Leu
                165                 170                 175

Thr Ala Val Ala Leu Ala Arg Leu Ala Thr Arg Thr Arg Arg Pro Ser
            180                 185                 190

Tyr Tyr Tyr Leu Leu Ala Leu Thr Ala Ser Asp Ile Ile Ile Gln Val
        195                 200                 205

Val Ile Val Phe Ala Gly Phe Leu Leu Gln Gly Ala Val Leu Ala Arg
    210                 215                 220

Gln Val Pro Gln Ala Val Val Arg Thr Ala Asn Ile Leu Glu Phe Ala
225                 230                 235                 240

Ala Asn His Ala Ser Val Trp Ile Ala Ile Leu Leu Thr Val Asp Arg
                245                 250                 255

Tyr Thr Ala Leu Cys His Pro Leu His His Arg Ala Ala Ser Ser Pro
            260                 265                 270

Gly Arg Thr Arg Arg Ala Ile Ala Ala Val Leu Ser Ala Ala Leu Leu
        275                 280                 285

Thr Gly Ile Pro Phe Tyr Trp Trp Leu Asp Met Trp Arg Asp Thr Asp
    290                 295                 300

Ser Pro Arg Thr Leu Asp Glu Val Leu Lys Trp Ala His Cys Leu Thr
305                 310                 315                 320

Val Tyr Phe Ile Pro Cys Gly Val Phe Leu Val Thr Asn Ser Ala Ile
                325                 330                 335

Ile His Arg Leu Arg Arg Arg Gly Arg Ser Gly Leu Gln Pro Arg Val
            340                 345                 350

Gly Lys Ser Thr Ala Ile Leu Leu Gly Ile Thr Thr Leu Phe Thr Leu
        355                 360                 365

Leu Trp Ala Pro Arg Val Phe Val Met Leu Tyr His Met Tyr Val Ala
    370                 375                 380

Pro Val His Arg Asp Trp Arg Val His Leu Ala Leu Asp Val Ala Asn
385                 390                 395                 400

Met Val Ala Met Leu His Thr Ala Ala Asn Phe Gly Leu Tyr Cys Phe

405                 410                 415

Val Ser Lys Thr Phe Arg Ala Thr Val Arg Gln Val Ile His Asp Ala
            420                 425                 430

Tyr Leu Pro Cys Thr Leu Ala Ser Gln Pro Glu Gly Met Ala Ala Lys
        435                 440                 445

Pro Val Met Glu Pro Pro Gly Leu Pro Thr Gly Ala Glu Val
    450                 455                 460

<210>  2
<211>  1389
<212>  DNA
<213>  Homo sapiens

<400>  2
atgagtatta tgatgttgcc catggagcaa aagatccagt gggtccccac ttccctgcag      60

gacatcactg ctgtcctggg tacagaagca tatactgagg aagacaaatc aatggtgtcc     120

catgcacaga aaagccagca ttcttgtctc agccattcca ggtggctgag gtctccacag     180

gtcacagggg gaagctggga cctccgaata aggccatcca aggactccag cagtttccgc     240

caggctcagt gtctgcgtaa ggatcctggg gcaaacaacc acttggagag ccaaggggtg     300

agaggtacag ctggcgatgc tgacagggag ctgcggggac cctcagaaaa agccacagct     360

ggccagccac gagtgaccct gctgcccacg ccccacgtca gcgggctgag ccaggagttt     420

gaaagccact ggccagagat cgcagagagg tccccgtgtg tggctggcgt catccctgtc     480

atctactaca gtgtcctgct gggcttgggg ctgcctgtca gcctcctgac cgcagtggcc     540

ctggcgcgcc ttgccaccag gaccaggagg ccctcctact actaccttct ggcgctcaca     600

gcctcggata tcatcatcca ggtggtcatc gtgttcgcgg gcttcctcct gcagggagca     660

gtgctggccc gccaggtgcc ccaggctgtg gtgcgcacgg ccaacatcct ggagtttgct     720

gccaaccacg cctcagtctg gatcgccatc ctgctcacgg ttgaccgcta cactgccctg     780

tgccaccccc tgcaccatcg ggccgcctcg tccccaggcc ggacccgccg ggccattgct     840

gctgtcctga gtgctgccct gttgaccggc atccccttct actggtggct ggacatgtgg     900

agagacaccg actcacccag aacactggac gaggtcctca agtgggctca ctgtctcact     960

gtctatttca tcccttgtgg cgtgttcctg gtcaccaact cggccatcat ccaccggcta    1020

cggaggaggg gccggagtgg gctgcagccc cgggtgggca agagcacagc catcctcctg    1080

ggcatcacca cactgttcac cctcctgtgg gcgccccggg tcttcgtcat gctctaccac    1140

atgtacgtgg cccctgtcca ccgggactgg agggtccacc tggccttgga tgtggccaat    1200

atggtggcca tgctccacac ggcagccaac ttcggcctct actgctttgt cagcaagact    1260

ttccgggcca ctgtccgaca ggtcatccac gatgcctacc tgccctgcac tttggcatca     1320

cagccagagg gcatggcggc gaagcctgtg atggagcctc cgggactccc cacaggggca     1380

gaagtgtag                                                             1389


<210>  3
<211>  23
<212>  DNA
<213>  Artificial

<220>
<223>  FLG Forward primer

<400>  3
gctgaaggaa cttctggaaa agg                                              23


<210>  4
<211>  24
<212>  DNA
<213>  Artificial

<220>
<223>  FLG Reverse primer

<400>  4
gttgtggtct atatccaagt gatc                                             24


<210>  5
<211>  22
<212>  DNA
<213>  Artificial

<220>
<223>  GAPDH Forward primer

<400>  5
gtctcctctg acttcaacag cg                                               22


<210>  6
<211>  22
<212>  DNA
<213>  Artificial

<220>
<223>  GAPDH Reverse primer

<400>  6
accaccctgt tgctgtagcc aa                                               22


<210>  7
<211>  22
<212>  DNA
<213>  Artificial

<220>
<223>  GPR142 Forward primer

<400>  7
cttgtctcag ccattccagg tg                                                22

<210>  8
<211>  22
<212>  DNA
<213>  Artificial

<220>
<223>  GPR142 Reverse primer

<400>  8
gatccttacg cagacactga gc                                                22

<210>  9
<211>  23
<212>  DNA
<213>  Artificial

<220>
<223>  GPR22 Forward primer

<400>  9
ccacacaaca tgaggctaca gac                                               23

<210>  10
<211>  22
<212>  DNA
<213>  Artificial

<220>
<223>  GPR22 Reverse primer

<400>  10
ctttctcgtc gttcacggtg tc                                                22

<210>  11
<211>  25
<212>  RNA
<213>  Artificial

<220>
<223>  siRNA sequence of GPR142-1(#HSS139493): Sense Sequence

<400>  11
ggcgucaucc cugucaucua cuaca                                             25

<210>  12
<211>  20
<212>  RNA
<213>  Artificial

<220>
<223>  siRNA sequence of GPR142-1(#HSS139493): Antisense Sequence

<400>  12
uguaguagau gacagggaug                                                   20

<210>  13
<211>  25
<212>  RNA
<213>  Artificial

<220>
<223>  siRNA sequence of GPR142-2(#HSS139494): Sense Sequence

<400>  13
ccaacauccu ggaguuugcu gccaa                                          25


<210>  14
<211>  22
<212>  RNA
<213>  Artificial

<220>
<223>  siRNA sequence of GPR142-2(#HSS139494): Antisense Sequence

<400>  14
uuggcagcaa acuccaggau gu                                             22


<210>  15
<211>  25
<212>  RNA
<213>  Artificial

<220>
<223>  siRNA sequence of GPR142-3(#HSS179846): Sense Sequence

<400>  15
gcuguccuga gugcugcccu guuga                                          25


<210>  16
<211>  22
<212>  RNA
<213>  Artificial

<220>
<223>  siRNA sequence of GPR142-3(#HSS179846): Antisense Sequence

<400>  16
ucaacagggc agcacucagg ac                                             22


<210>  17
<211>  25
<212>  RNA
<213>  Artificial

<220>
<223>  siRNA sequence of Control-1(Low GC, #129350-200): Sense Sequence

<400>  17
ggaugucaac uugagcaacu uauuu                                          25


<210>  18
<211>  25

<212>  RNA
<213>  Artificial

<220>
<223>  siRNA sequence of Control-1(Low GC, #129350-200): Antisense
       Sequence

<400>  18
aaauaaguug cucaaguuga caucc                                          25


<210>  19
<211>  25
<212>  RNA
<213>  Artificial

<220>
<223>  siRNA sequence of Control -2(MED GC, #129350-300): Sense Sequence

<400>  19
gguaggugag uguacagacg caaua                                          25


<210>  20
<211>  25
<212>  RNA
<213>  Artificial

<220>
<223>  siRNA sequence of Control -2(MED GC, #129350-300): Antisense
       Sequence

<400>  20
uauugcgucu guacacucac cuacc                                          25


<210>  21
<211>  25
<212>  RNA
<213>  Artificial

<220>
<223>  siRNA sequence of Control-3(HIGH GC, #129350-400): Sense Sequence

<400>  21
ggcggcucgu gcaacacagg cgcuu                                          25


<210>  22
<211>  25
<212>  RNA
<213>  Artificial

<220>
<223>  siRNA sequence of Control-3(HIGH GC, #129350-400): Antisense
       Sequence

<400>  22
aagcgccugu guugcacgag ccgcc                                          25


<210>  23
<211>  25

<212>  RNA
<213>  Artificial

<220>
<223>  siRNA sequence of GPR22-1(#HSS104365): Sense Sequence

<400>  23
uagucauggc uuauggaaca acuau                                          25


<210>  24
<211>  25
<212>  RNA
<213>  Artificial

<220>
<223>  siRNA sequence of GPR22-1(#HSS104365): Antisense Sequence

<400>  24
auaguuguuc cauaagccau gacua                                          25


<210>  25
<211>  25
<212>  RNA
<213>  Artificial

<220>
<223>  siRNA sequence of GPR22-2(#HSS104367): Sense Sequence

<400>  25
gaguuguuuc uauaguagaa gcuga                                          25


<210>  26
<211>  25
<212>  RNA
<213>  Artificial

<220>
<223>  siRNA sequence of GPR22-2(#HSS104367): Antisense Sequence

<400>  26
ucagcuucua cuauagaaac aacuc                                          25


<210>  27
<211>  25
<212>  RNA
<213>  Artificial

<220>
<223>  siRNA sequence of GPR22-3(#HSS178711): Sense Sequence

<400>  27
ggcagagcug uaauguuaau gauau                                          25


<210>  28
<211>  25
<212>  RNA
<213>  Artificial

<220>
<223>  siRNA sequence of GPR22-3(#HSS178711): Antisense Sequence

<400>  28
auaucauuaa cauuacagcu cugcc                                          25

## Description

### Technical Field

The present invention relates to a filaggrin (FLG) production promoter including a certain G protein-coupled receptor (GPR) agonist as an active ingredient and to a therapeutic agent for a disease associated with reduced filaggrin production, and to a screening method for the therapeutic agent.

### Background Art

Stratum corneum, which is located in the outermost layer of epidermis and includes as several layers to dozens of layers laminated with regularity, functions as a barrier layer against invasion of substances and allergens from the outside, ultra-violet rays, and the like. In addition, the stratum corneum has a suppressive action on water release from the body and a retaining action of water in the stratum corneum itself in order to protect the body from dryness of an external environment. As a factor having the water retaining action on the stratum corneum itself, a natural moisturizing factor (NMF) is known. Amino acids as main components of the NMF are known to have a high correlation with a water content, in particular, in the stratum corneum, and are extremely important for the water retention of the stratum corneum. The amino acids are produced by degradation of filaggrin derived from keratohyalin granules in the stratum corneum. Filaggrin is expressed as profilaggrin in epidermal keratinocytes, is immediately phosphorylated, and is accumulated in keratohyalin granules. The profilaggrin is a histidine-rich protein of about 400 KDa and is formed of 10 to 12 repeating units, each of which is filaggrin containing 324 amino acids (hereinafter also referred to simply as "FLG" or "FLG monomer"), which are tandemly bound. Thereafter, profilaggrin (pro-FLG) is subjected to dephosphorylation and hydrolysis and is degraded into FLG, and the FLG is transferred to the stratum corneum. In the stratum corneum, FLG increases the aggregation efficiency of keratin filaments, and is involved in the internal construction of corneocytes (J. Invest. Dermatol. (1994) 103(5): 731-740, J. Cell Science (2009) 122(9): 1285-1294). In these manners, FLG plays important roles in the barrier function of the skin.

There are many reports on relationships between FLG and skin diseases. For example, it has been reported that a gene mutation in FLG causes ichthyosis vulgaris and that the gene mutation in FLG is a major factor for the onset of atopic dermatitis (Nature Genetics (2006) 38: 337-342, Nature Genetics (2006) 38: 441-446, J. Allergy Clin. Immunol. (2007) 119(2): 434-44). Ichthyosis is a congenital skin disease characterized by abnormalities of keratogenesis in which the surface of the skin is hardened in a form of a fish scale and is peeled off. The failure of keratinization in epidermis weakens the moisture-retaining function of the skin and impairs the protective function against invasion of allergens and the like from the outside. In addition, it is reported that, in patients with of atopic dermatitis in Japan, about one third of the total patients has a gene mutation in FLG (British Journal of Dermatology (2009) 161: 1387-1390).

Atopic dermatitis is a highly frequent refractory allergic disease, which is observed in about 20% of children in developed countries. The cause of atopic dermatitis is still unknown and it is thought that a variety of factors such as environmental factors, food, psychological stress, and systemic and local infections are involved in addition to the genetic factor. Atopic dermatitis can be divided into a type in which the amount of IgE antibody is increased (extrinsic atopic dermatitis) and a normal type (intrinsic atopic dermatitis). Intrinsic atopic dermatitis is caused by a factor such as psychological stress, and in this type, there is no mutation in FLG gene in most cases. On the other hand, in extrinsic atopic dermatitis, the mutation in FLG gene as described above is associated in many cases.

The basis of the treatment of such atopic dermatitis is the combination of elimination of the onset and complicating factors and the medicinal treatment for correcting abnormal skin functions and for suppressing inflammation. In addition, examples of the main abnormalities of skin functions in atopic dermatitis include a reduced water retention ability and barrier function of the stratum corneum, a reduced itching threshold and susceptibility to infection.

The medicinal treatment for the reduced water retention ability and barrier function of the stratum corneum, the reduced itching threshold, and the susceptibility to infection varies depending on the subject of prescription, the timing of prescription, and the like. In general, itching includes peripheral itching in which histamine and itch-inducing substances produced by eosinophils are involved and central itching in which opioid receptors are involved. Medicaments for treating such itching are prescribed depending on the degrees of itching. The degrees of itching are classified into the stages of from "insensible itching" to "unbearable itching" to be diagnosed, and when itching of the unbearable degree occurs, an antipruritic agent is prescribed. In addition, when a patient with atopic dermatitis is diagnosed as being liable to develop infections of the skin, i.e., being susceptible to infections of the skin such as contagious impetigo, herpes simplex, and molluscum contagiosum, the medicament for each of these infections is prescribed. On the other hand, the medicament for the reduced water retention ability and barrier function of the stratum corneum is prescribed to a patient with atopic dermatitis with a reduced water retention ability and barrier function of the stratum corneum due to reduced FLG production, regardless of the degrees of itching and the timing of developing itching or the timing of developing infection.

It is known that the causes of reduced FLG production in atopic dermatitis include, in addition to the gene mutation in FLG, inclining of the balance of equilibrium of Th1 cells and Th2 cells to the dominant side for the Th2 cells for some reason, which causes the overproduction of Th2 cytokines such as IL-4 and IL-13, leading to reduced FLG expression, though there is no mutation in FLG gene (Non Patent Literature 1: J. Investigative Dermatology (2008) 128: 2248-2258).

Heretofore, as ingredients for promoting the production of FLG, there have been proposed naturally-derived ingredients such as chebulanins (Patent Literature 1: JP 2009-256269 A), liquiritin (Patent Literature 2: JP 2003-146886 A), glycolipids (Patent Literature 3: JP 2006-241095 A), rosmarinic acid, and eriodictyol-7-O-rutinoside (Patent Literature 4: JP 2010-90037 A), but it is desired to develop an additional safe and highly effective substance having an FLG production-promoting action, which can be used for the treatment/prevention of skin diseases such as atopic dermatitis. However, a target receptor for such substance is not known, and therefore, in the present circumstances, a substance having an FLG production-promoting action cannot be systematically screened.

G protein-coupled receptor 142 (hereinafter simply referred to as "GPR142") is an orphan G protein-coupled receptor (orphan GPR) belonging to the rhodopsin family. Human GPR142 is a protein formed of a sequence of 462 amino acids (FEBS Letters 554 (2003) 381-388). Murine Pgr (GPR142) is reported to be expressed in islets of Langerhans (Cell 135, 561-571, Supplemental Data (2008)). In addition, murine GPR142 mRNA is reported to be expressed in brain, spleen, liver, kidney, and testis (Neuropharmacology 50 (2006): 512-520). Further, Nutrition & Metabolism (2008) 5: 23 describes that the antisense knockdown of GPR142a in zebrafish led to an observation of a significant decrease in fat, and therefore, a GPR142 antagonist may be a candidate agent for the treatment of obesity. Further, 242nd ACS National Meeting & Exposition, Denver, CO, United States, August 28-September 1, 2011 (2011), MEDI-134 and MEDI-135 report that GPR142 is expressed in β cells of the pancreas and that phenylalanine derivatives and aminopyrazole phenylalanine as GPR142 agonists for the treatment of type II diabetes promote the secretion of insulin in a GPR142-dependent manner, and Patent Literature 5 (WO 2010/093849 A1) describes these compounds. However, as far as the inventors of the present invention know, there is no report that describes a relationship between GPR142 and an FLG-producing effect. As well, there is no report that describes that a GPR142 agonist promotes FLG production and is used for the treatment of skin diseases.

Patent Literature 6 (JP 2004-175687 A) describes that the skin of a hairless mouse was subjected to tape stripping to physically break the skin barrier, and thereafter, a cyclic AMP (cyclic AMP or cAMP) antagonist was applied to decrease the calcium level within the cells, resulting in restoration of the barrier. However, Patent Literature 6 does not describe that a cAMP antagonist restores the break of the skin barrier associated with reduced FLG production or that FLG production is promoted through GPR142.

Patent Literature 7 (WO 2002/074341 A1) describes that nociceptin antagonists such as N-quinolylbenzamide derivatives including N-(4-amino-2-methyl-6-quinolyl)-2-[(4-ethylphenoxy)methyl]benzamide are used as antipruritic agents for the prevention and treatment of itching caused by numerous diseases such as diseases associated with itching, local pruritus cutaneous, and nodular prurigo. In addition, Patent Literature 7 discloses atopic dermatitis as an example of the numerous diseases associated with itching. However, Patent Literature 7 does not describe that an N-quinolylbenzamide derivative has an FLG production-producing effect, does not describe anything about GPR142, and does not describe or suggest that the N-quinolylbenzamide derivative is expected to be able to be used as a medicament for treating skin diseases such as atopic dermatitis by alleviating the reduced water retention ability and barrier function of the skin. Further, Patent Literature 7 does not disclose test examples specifically using a model of itching caused by atopic dermatitis.

Patent Literature 8 (JP 3013989 B2, JP 11-335355 A) discloses N-quinolylbenzamide derivatives including N-(4-amino-2-methyl-6-quinolyl)-2-[(4-ethylphenoxy)methyl]benzamide as nociceptin antagonists and describes an analgesic as an example of the applications thereof, but does not describe anything about GPR142 or an FLG production-promoting effect.

Ivermectin is commercially available under a trade name of Stromectol as a therapeutic agent for intestinal strongyloidiasis and sarcoptic mange. In addition, it is reported that ivermectin has an effect on a mouse model of allergic asthma (Inflammation Research (2011) 60(6): 589-596), but there is no description about GPR142 or an FLG production-promoting effect.

### Citation List

### Patent Literature

[PTL 1] JP 2009-256269 A
[PTL 2] JP 2003-146886 A
[PTL 3] JP 2006-241095 A
[PTL 4] JP 2010-90037 A
[PTL 5] WO 2010/093849 A1
[PTL 6] JP 2004-175687 A
[PTL 7] WO 2002/074341 A1
[PTL 8] JP 3013989 B2, JP 11-335355 A

### Non Patent Literature

[NPL 1] J. Investigative Dermatology (2008) 128: 2248-2258

### Summary of Invention

### Technical Problem

An object of the present invention is to provide a filaggrin (FLG) production promoter and a therapeutic agent useful for the treatment of a disease, in particular, a skin disease such as atopic dermatitis associated with reduced FLG production. Another object of the present invention is to provide a screening method for a substance useful as the therapeutic agent.

### Solution to Problem

In order to achieve the above-mentioned objects, the inventors of the present invention have eagerly searched an FLG production promoter and a target receptor for the FLG production promoter, and as a result, have found that FLG production is promoted through a certain G protein-coupled receptor (GPR). In addition, the present inventors have found that a compound having an agonist action on the certain GPR promotes FLG production in the skin and satisfactorily improves a water retention ability and/or barrier function of the stratum corneum, and thus completed the present invention.

That is, the present invention includes the following.
1. A therapeutic agent for a disease associated with reduced filaggrin (FLG) production, including a GPR142 agonist as an active ingredient.
2. A therapeutic agent for a disease according to the above-mentioned item 1, in which the disease associated with reduced FLG production includes a skin disease.
3. A therapeutic agent for a disease according to the above-mentioned item 2, in which the skin disease includes atopic dermatitis.
4. A therapeutic agent for a disease according to the above-mentioned item 2, in which the skin disease includes ichthyosis vulgaris.
5. A therapeutic agent for a disease according to any one of the above-mentioned items 1 to 4, in which the GPR142 agonist includes N-(4-amino-2-methyl-6-quinolyl)-2-[(4-ethylphenoxy)methyl]benzamide or a pharmaceutically acceptable salt thereof.
6. A therapeutic agent for a disease according to any one of the above-mentioned items 1 to 4, in which the GPR142 agonist is a compound represented by the following general formula (I) or a pharmaceutically acceptable salt thereof: 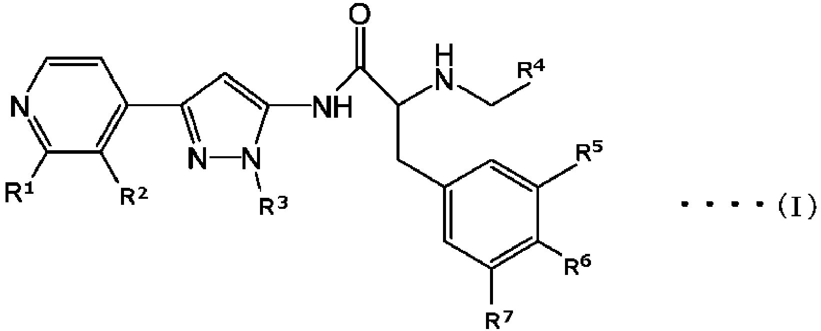 where R¹ and R² each represent a hydrogen atom, a halogen atom, or an alkyl group having 1 to 3 carbon atoms, R³ represents an alkyl group having 1 to 3 carbon atoms, R⁴ represents an alkoxycarbonyl group having 1 to 3 carbon atoms or a hydroxycarbonyl group, and R⁵, R⁶, and R⁷ each represent a hydrogen atom or a halogen atom.
7. A therapeutic agent for a disease according to the above-mentioned item 6, in which, in the general formula (I) as described in the above-mentioned item 6, R¹ and R² each represent a hydrogen atom, R³ represents a methyl group, R⁴ represents an ethoxycarbonyl group or a hydroxycarbonyl group, and R⁵, R⁶, and R⁷ each represent a hydrogen atom.
8. A therapeutic agent for a disease according to any one of the above-mentioned items 1 to 4, in which the GPR142 agonist includes ivermectin.
9. A therapeutic agent for a disease associated with reduced FLG production for alleviating a symptom of the disease associated with reduced FLG production, the therapeutic agent for a disease associated with reduced FLG production including a GPR142 agonist as an active ingredient.
10. A therapeutic agent for a disease according to the above-mentioned item 9, in which the disease associated with reduced FLG production includes a skin disease.
11. A therapeutic agent for atopic dermatitis for alleviating a reduced water retention ability and/or barrier function of stratum corneum of skin associated with atopic dermatitis, the therapeutic agent for atopic dermatitis including a GPR142 agonist as an active ingredient.
12. An FLG production promoter, including a GPR142 agonist as an active ingredient.
13. A therapeutic method for a disease associated with reduced FLG production, including administering an effective amount of a medicament including a GPR142 agonist as an active ingredient to a patient presenting a symptom of a disease associated with reduced FLG production to alleviate the symptom of the patient.
14. A therapeutic method for atopic dermatitis, including administering an effective amount of a medicament including a GPR142 agonist as an active ingredient to a patient with atopic dermatitis with reduced FLG production to alleviate a stratum corneum abnormality of skin associated with atopic dermatitis of the patient.
15. A screening method for a therapeutic agent for a disease associated with reduced FLG production, including evaluating whether a test substance is capable of promoting an activity or expression of GPR142 or not.
16. A screening method according to the above-mentioned item 15, in which the screening method includes the following steps (a) to (c):
   (a) bringing a GPR142-expressing cell or a membrane fraction thereof into contact with a test substance;
   (b) measuring an activity or expression of GPR142 in the cell or membrane fraction thereof after the contact with the test substance; and
   (c) selecting a substance capable of promoting the activity or expression of GPR142 in the cell or membrane fraction thereof.
17. A screening method according to the above-mentioned item 15 or 16, in which the disease associated with reduced FLG production is a skin disease.
18. A screening method according to the above-mentioned item 17, in which the skin disease is atopic dermatitis.
19. A screening method according to any one of the above-mentioned items 15 to 18, in which the GPR142-expressing cell includes a cell obtained by transformation with an expression vector including a GPR142 gene.
20. A screening method according to the above-mentioned item 19, in which the GPR142 gene includes a human GPR142 gene having an amino acid sequence set forth in SEQ ID NO: 1 or a gene encoding a functional variant of human GPR142 that has an amino acid sequence with 90% or more identity to the amino acid sequence and retains an activity of GPR142.

Further, other aspects of the present invention include the following.
(A) A pharmaceutical composition for promoting FLG production, including a GPR142 agonist or a pharmaceutically acceptable salt thereof and a pharmaceutically acceptable excipient.
(B) A pharmaceutical composition for alleviating a stratum corneum abnormality, including a GPR142 agonist or a pharmaceutically acceptable salt thereof and a pharmaceutically acceptable excipient.
(C) A pharmaceutical composition for moisturizing skin, including a GPR142 agonist or a pharmaceutically acceptable salt thereof and a pharmaceutically acceptable excipient.
(D) A pharmaceutical composition for improving a barrier function of skin, including a GPR142 agonist or a pharmaceutically acceptable salt thereof and a pharmaceutically acceptable excipient.
(E) A pharmaceutical composition according to any one of the above-mentioned items (A) to (D), in which the pharmaceutical composition is used for preventing or treating atopic dermatitis.
(F) A pharmaceutical composition according to any one of the above-mentioned items (A) to (D), in which the pharmaceutical composition is used for preventing or treating ichthyosis vulgaris as a disease.
(G) A pharmaceutical composition for preventing or treating a disease associated with reduced FLG production, including a GPR142 agonist or a pharmaceutically acceptable salt thereof and a pharmaceutically acceptable excipient.
(H) A pharmaceutical composition for preventing or treating a disease associated with a stratum corneum abnormality, including a GPR142 agonist or a pharmaceutically acceptable salt thereof and a pharmaceutically acceptable excipient.
(I) A pharmaceutical composition for preventing or treating a disease associated with a reduced water retention ability of skin, including a GPR142 agonist or a pharmaceutically acceptable salt thereof and a pharmaceutically acceptable excipient.
(J) A pharmaceutical composition for preventing or treating a disease associated with a reduced barrier function of skin, including a GPR142 agonist or a pharmaceutically acceptable salt thereof and a pharmaceutically acceptable excipient.
(K) A pharmaceutical composition according to any one of the above-mentioned items (G) to (J), in which the disease includes atopic dermatitis.
(L) A pharmaceutical composition according to any one of the above-mentioned items (G) to (J), in which the disease includes ichthyosis vulgaris.
(M) A GPR142 agonist or pharmaceutically acceptable salt thereof for preventing or treating a disease associated with reduced FLG production.
(N) A GPR142 agonist or pharmaceutically acceptable salt thereof for preventing or treating a disease associated with a stratum corneum abnormality.
(O) A GPR142 agonist or pharmaceutically acceptable salt thereof for preventing or treating a disease associated with a reduced water retention ability of skin.
(P) A GPR142 agonist or pharmaceutically acceptable salt thereof for preventing or treating a disease associated with a reduced barrier function of skin.
(Q) A compound or salt thereof according to any one of the above-mentioned items (M) to (P), in which a subject of the prevention or treatment is included in a patient with atopic dermatitis.
(R) A compound or salt thereof according to any one of the above-mentioned items (M) to (P), in which a subject of the prevention or treatment is included in a patient with ichthyosis vulgaris.
(S) A method of promoting FLG production, including administering an effective amount of a GPR142 agonist or a pharmaceutically acceptable salt thereof to a subject.
(T) A method of preventing or treating a disease associated with a stratum corneum abnormality, administering an effective amount of a GPR142 agonist or a pharmaceutically acceptable salt thereof to a subject.
(U) A method of preventing or treating a disease associated with a reduced water retention ability of skin, including administering an effective amount of a GPR142 agonist or a pharmaceutically acceptable salt thereof to a subject.
(V) A method of preventing or treating a disease associated with a reduced barrier function of skin, including administering an effective amount of a GPR142 agonist or a pharmaceutically acceptable salt thereof to a subject.
(W) A method according to any one of the above-mentioned items (S) to (V), in which the subject includes a patient with atopic dermatitis associated with reduced FLG production.
(X) A method according to any one of the above-mentioned items (S) to (V), in which the subject includes a patient with ichthyosis vulgaris.

### Advantageous Effects of Invention

It was confirmed that the GPR142 agonist according to the present invention promoted FLG production in the skin, satisfactorily improved a water retention ability and barrier function of the stratum corneum, and acted as an active ingredient of an FLG production promoter. In addition, the GPR142 agonist according to the present invention can act as an active ingredient of a therapeutic agent for a disease, in particular, a skin disease such as atopic dermatitis associated with reduced FLG production. Further, by targeting GPR142, the therapeutic agent for a disease associated with reduced FLG production can be systematically screened.

### Brief Description of Drawings

FIGS. 1 are graphs confirming transcription-activating effects on an FLG promoter of (a) Compound 1 and Compound 1+calcium, (b) Comparative Compound 1 and Comparative Compound 1+calcium, and (c) Compound 1 and Comparative Compound 2 by luciferase assay (Example 1).
FIG. 2 is a graph confirming expression-increasing effects on FLG mRNA of Compound 1 and Compound 2 by real-time PCR in NHEK cells with or without pretreatment with calcium (Example 2).
FIG. 3 is a graph confirming mRNA expression of GPR142 and GPR22 in NHEK cells by Compound 1 or Compound 1+calcium treatment by real-time PCR (Example 4).
FIGS. 4 are graphs confirming expression amounts of GPR142 mRNA and FLG mRNA in NHEK cells by Compound 1 or Compound 1+calcium treatment in the presence of GPR142 RNAi by real-time PCR (Example 5).
FIGS. 5 are graphs confirming expression amounts of GPR142 mRNA and FLG mRNA in NHEK cells by Compound 1, Compound 2, or Compound 3 treatment in the presence of GPR142 RNAi by real-time PCR (Example 6).
FIGS. 6 are graphs confirming expression amounts of GPR22 mRNA and FLG mRNA in NHEK cells by Compound 1 or Compound 1+calcium treatment in the presence of GPR22 RNAi by real-time PCR (Reference Example 1).
FIGS. 7 are graphs confirming FLG mRNA and GPR142 mRNA expression by Compound 1 in GPR142-overexpressing NHEK cells by real-time PCR (Example 7).
FIG. 8 is a graph confirming an action of Compound 1 on an adenylate cyclase activity in GPR142-overexpressing HEK293T cells (Example 8).
FIG. 9 is a graph confirming an action of Compound 2, Compound 3, or Comparative Compound 2 on an adenylate cyclase activity in GPR142-overexpressing HEK293T cells (Example 9).
FIG. 10 is a graph confirming the expression-increasing effect on FLG mRNA of Compound 1 or Comparative Compound 2 in three-dimensional cultured skin by real-time PCR (Example 10).
FIG. 11 is a photograph confirming the production ability of FLG monomer by Compound 1, Comparative Compound 2, or Comparative Compound 3 in three-dimensional cultured skin by immunoblotting (Example 11).
FIGS. 12 are photographs confirming the production state of FLG protein by Compound 1 in human normal skin by immunohistostaining (Example 12).
FIG. 13 is a photograph confirming the production ability of FLG monomer by immunoblotting when Compound 1 was administered to FLG gene-deficient homozygous and heterozygous mouse models (Example 13).
FIG. 14 is a graph showing the time course of clinical manifestations when Compound 1 was administered to a mouse model developing atopic dermatitis (NC/Nga) (Example 14, (i)).
FIGS. 15 are graphs showing results of the measurement of a transepidermal water loss (TEWL) when Compound 1 was administered to a mouse model developing atopic dermatitis (NC/Nga). FIG. 15(a) shows the results at 8 weeks after the beginning of administration and FIG. 15(b) shows the results at 12 weeks after the beginning of administration (Example 14, (ii)).
FIGS. 16 are photographs showing the appearance of mice at 12 weeks after the beginning of administration of Compound 1 to mouse models developing atopic dermatitis (NC/Nga). FIG. 16(a) shows the result of a control mouse and FIG. 16(b) shows the result of a mouse that received Compound 1 (Example 14, (iii)).
FIGS. 17 are a photograph and graph showing the results of FLG monomer production ability of atopic dermatitis mouse models (NC/Nga) that received Compound 1 confirmed by immunoblotting (Example 15).

### Description of Embodiments

As described in Examples below, the inventors of the present invention found for the first time that FLG production is promoted via GPR142, which is one of the G protein-coupled receptors (GPRs). Herein, a substance capable of promoting FLG production via GPR142-dependent intracellular signal transduction is referred to as a "GPR142 agonist". The GPR142 agonist is useful as an FLG production promoter and can be used for a therapeutic agent for a disease associated with reduced FLG production.

In addition, FLG production is promoted via GPR142, and hence an FLG production promoter can be systematically screened by identifying a GPR142 agonist by using the GPR142 as a target receptor. The identified GPR142 agonist is useful as a therapeutic agent for a disease associated with reduced FLG production.

The present invention relates to a medicament such as an FLG production promoter and a therapeutic agent for a disease associated with reduced FLG production, both of which include a GPR142 agonist as an active ingredient. The GPR142 agonist, which is the active ingredient of the medicament according to the present invention, acts on GPR142 and can promote the production of FLG mRNA and FLG protein through the receptor. The GPR142 agonist, which is the active ingredient of the medicament according to the present invention, can preferably promote the production of FLG protein (i.e., FLG monomer). The present invention also encompasses a method of promoting FLG production by bringing a GPR142 agonist into contact with a cell (preferably a skin cell).

In the present invention, an example of the GPR142 agonist is N-(4-amino-2-methyl-6-quinolyl)-2-[(4-ethylphenoxy)methyl]benzamide or a pharmaceutically acceptable salt thereof.

In the present invention, further examples of the GPR142 agonist include phenylalanine derivatives or aminopyrazole phenylalanine or pharmaceutically acceptable salts thereof as described in Patent Literature 5 (WO 2010/093849 A1). Specifically, for example, a compound represented by the following general formula (I) or a pharmaceutically acceptable salt thereof is preferred. 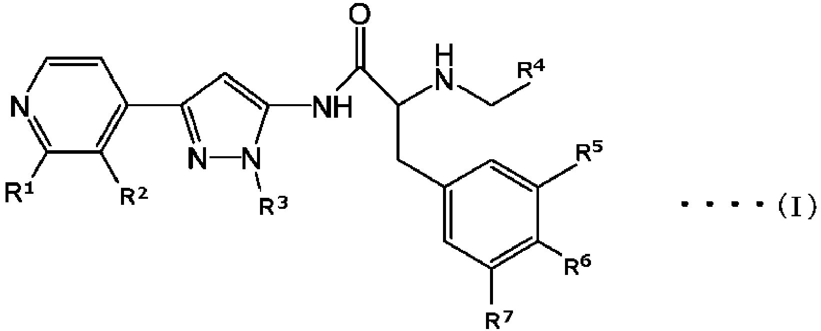 [In the formula, R¹ and R² each represent a hydrogen atom, a halogen atom, or an alkyl group having 1 to 3 carbon atoms, R³ represents an alkyl group having 1 to 3 carbon atoms, R⁴ represents an alkoxycarbonyl group having 1 to 3 carbon atoms or a hydroxycarbonyl group, and R⁵, R⁶, and R⁷ each represent a hydrogen atom or a halogen atom.]

In the general formula (I), R¹ and R² each preferably represent a hydrogen atom. In addition, R³ preferably represents a methyl group. R⁴ preferably represents a methoxycarbonyl group, an ethoxycarbonyl group, or a hydroxycarbonyl group. R⁵, R⁶, and R⁷ each preferably represent a hydrogen atom. Most preferred examples of the compound represented by the general formula (I) include methyl 2-(1-(1-methyl-3-(pyridin-4-yl)-1H-pyrazol-5-ylamino)-1-oxo-3-phenylpropan-2-ylamino)acetate dihydrochloride, ethyl 2-(1-(1-methyl-3-(pyridin-4-yl)-1H-pyrazol-5-ylamino)-1-oxo-3-phenylpropan-2-ylamino)acetate dihydrochloride, and 2-(1-(1-methyl-3-(pyridin-4-yl)-1H-pyrazol-5-ylamino)-1-oxo-3-phenylpropan-2-ylamino)acetic acid dihydrochloride.

A further example of the GPR142 agonist in the present invention is ivermectin. A specific example thereof is a mixture of 90% or more of 22,23-dihydroavermectin B1a and 10% or less of 22,23-dihydroavermectin Bib. In addition to those agonists, agonists described in EP 0001 689 B1 and US 2010/0004200 A1 may also be used.

Herein, the "pharmaceutically acceptable salt thereof" may be any salt as long as the salt can be used for a pharmaceutical and forms a salt with the GPR142 agonist, which is the active ingredient of the therapeutic agent for a disease according to the present invention, for example, the compound represented by the general formula (I), and such salt can be obtained through a reaction, for example, with an inorganic acid such as hydrochloric acid, sulfuric acid, or phosphoric acid, hydrobromic acid; or an organic acid such as oxalic acid, malonic acid, citric acid, fumaric acid, maleic acid, lactic acid, malic acid, succinic acid, tartaric acid, mandelic acid, acetic acid, propionic acid, gluconic acid, ascorbic acid, methanesulfonic acid, or benzenesulfonic acid. In the present invention, a hydrochloride is preferred. It should be noted that the present invention also encompasses a hydrated compound or hydrate, solvate, and crystalline polymorphic form of each compound.

In addition, a prodrug and metabolite of each compound that is the GPR142 agonist according to the present invention are also encompassed. The term "prodrug" is a derivative of the compound according to the present invention, which has a chemically or metabolically degradable group and exhibits the original drug efficacy by returning to the original compound after being administered to the living body, and examples of the prodrug include a complex not formed by a covalent bond and a salt.

In the present invention, when the GPR142 agonist is used as a pharmaceutical preparation, the agonist may be generally used as a mixture with, for example, a carrier, an excipient, a diluent, an extender, a disintegrant, a stabilizing agent, a preservative, a buffer, an emulsifying agent, a fragrance, a colorant, a sweetener, a thickener, a taste masking agent, a solubilizing aid, and any other additive, which are pharmaceutically acceptable and known per se. Specific examples thereof include water, a vegetable oil, an alcohol such as ethanol or benzyl alcohol, polyethylene glycol, glycerol triacetate, gelatin, lactose, a carbohydrate such as starch, magnesium stearate, talc, lanolin, and vaseline. Examples of the pharmaceutical preparation include a liquid, a lotion, a suspension, an emulsion, an ointment, a gel, a capsule, a tablet, a sugar-coated tablet, a granule, and a suppository. An adjuvant, a stabilizing agent, a moisturizing or emulsifying agent, a buffer, and any other commonly used additive may be blended in such preparation as desired.

The GPR142 agonist, which is the active ingredient of the therapeutic agent according to the present invention, has an FLG production-promoting action, and hence is used for a therapeutic agent for a disease, preferably a skin disease associated with reduced FLG production. Examples of the skin disease associated with reduced FLG production include atopic dermatitis, ichthyosis vulgaris, metal (nickel) allergic contact dermatitis (J. Investigative Dermatology (2008) 128: 1430-1435), and asteatotic eczema (senile xerosis) (Arch Dermatol Res. (2004) 295: 448-452). However, the skin diseases are not limited to the above-mentioned diseases as long as the skin diseases are associated with reduced FLG production.

As described in the section entitled Background Art in detail, examples of major dysfunctions of the skin in atopic dermatitis are a reduced water retention ability and barrier function of the stratum corneum, a reduced itching threshold, and susceptibility to infection, and the GPR142 agonist according to the present invention is useful as a therapeutic agent for atopic dermatitis particularly for alleviating a reduced water retention ability and/or barrier function of the stratum corneum in the skin associated with atopic dermatitis.

Herein, the stratum corneum abnormality refers to a condition in which a water retention ability, i.e., a moisture-retaining function of the stratum corneum is impaired and a condition in which a barrier function of the stratum corneum is reduced and, as a result, a protective function against the invasion of allergens and the like form the outside is impaired.

The stratum corneum acts as a boundary to the external environment and has functions such as a protective function of the internal parts of the body from the external environment (an action of adjusting the moisture release from the body to protect the body from the external environment such as dryness and a water retaining action of the stratum corneum itself) and a function of retaining internal substances of the body (an action as a barrier layer against the invasion of substances and allergens from the outside and against ultra-violet rays and the like). For example, in atopic dermatitis, dry skin (dryness) associated with papules colocalized with pores is observed even in areas without skin inflammation, and the amount of water that transpires from the surface of the skin per hour per square meter of the body surface area, i.e., transepidermal water loss (TEWL) measured in a patient with atopic dermatitis is higher than that in a healthy individual (about 7.4 g/h·m²). Accordingly, in the present invention, the water retention ability and/or barrier function of the stratum corneum can be confirmed by measuring the transepidermal water loss and the degree of dry skin (dryness).

The water retention ability and barrier function of the skin of a human can be measured according to the method described below. The water retention ability can be measured, for example, according to the method described in J. Dermatology (2011) 38: 685-692. Specific procedures of the method are as described below. A subject is left at rest in a room at a temperature of 21°C and a humidity of 50±5% for 20 minutes. During this period, the subject is made to wear a test T-shirt. At five minutes after taking off the test T-shirt, the test T-shirt is subjected to the measurement of a transepidermal water loss (TEWL) with a water transpiration measuring instrument, or alternatively, a skin conductance (stratum corneum water content: SCWC) is measured with a water content measuring instrument for the stratum corneum. The measurement of the barrier function can be conducted, for example, according to the method described in J. Dermatology (2011) 38: 685-692. Specifically, (i) the degree of dry skin (dryness) can be diagnosed on a five-point scale by means of visual inspection (the degree of dry skin: (0) null (none); (1) modest (slightly rough); (2) moderate (rough); (3) severe (apparently rough with peeling of the stratum corneum); and (4) significant (marked)), or alternatively, (ii) the change in the stratum corneum is observed by dermoscopy and the stratum corneum abnormality can be diagnosed on a five-point scale ((0) null (smooth); (1) modest (slightly rough); (2) moderate (rough); (3) severe (apparently rough with peeling of the stratum corneum); and (4) significant (marked)).

In addition, the reduced FLG production can be determined by measuring an amount of FLG mRNA in the skin (for example, by a PCR method) or an amount of FLG protein in the skin (for example, by western blotting). Alternatively, the degree of the reduced FLG production is correlated with the amount of water transpiration and the degree of dry skin (J. Invest. Dermatol. (2009) 129(3): 682-689), the reduced FLG production can be determined by evaluating the degree of dry skin (dryness) by the above-mentioned method for measuring the amount of water transpiration or by means of visual inspection and the like, and the method of determining the reduced FLG production by evaluating the degree of dry skin (dryness) by means of visual inspection is simple and convenient.

A method of administering the medicament according to the present invention is not particularly limited and is appropriately determined depending on the purpose of the administration, and examples of the method include oral, intraarterial, intravenous, intraperitoneal, subcutaneous, intradermal, percutaneous, and transmucosal administrations. The dosage form of the medicament according to the present invention is not particularly limited and is appropriately determined depending on the respective purposes. In the case of oral administration of the medicament according to the present invention including a GPR142 agonist as an active ingredient, for example, the daily dose falls within the range of from 0.001 to 100 mg/kg, preferably of from 0.1 to 30 mg/kg and the medicament can be administered once a day to a plurality of times per day, or alternatively, the medicament can be administered once per 2 to 4 days. In the case of intravenous administration, the suitable daily dose is from about 0.0001 to 10 mg/kg and the medicament can be administered once a day to a plurality of times per day. In addition, in the case of a percutaneous agent, the dose of from about 0.001 to 100 mg/kg body weight can be administered once a day to a plurality of times per day. The dose can be appropriately determined depending on individual cases considering the symptom, age, sex, and the like. The therapeutic agent according to the present invention promotes FLG production as described in Examples below. In addition, the therapeutic agent according to the present invention alleviates the stratum corneum abnormality (reduced water retention ability and/or barrier function) of the skin associated with atopic dermatitis.

The present invention also encompasses a screening method for a therapeutic agent for a disease associated with reduced FLG production. As described in Examples below, FLG production is promoted via GPR142-dependent intracellular signal transduction, and hence an FLG production promoter can be systematically screened by identifying a GPR142 agonist by using GPR142 as a target receptor. The GPR142 agonist identified by the screening method according to the present invention is useful as a therapeutic agent for a disease associated with reduced FLG production.

The screening method for a therapeutic agent for a disease associated with reduced FLG production according to the present invention includes a step of evaluating whether a substance is capable of promoting an activity or expression of GPR142 or not, and can be conducted by using, for example, a cell expressing GPR142 (also referred to as a "GPR142-expressing cell").

The "GPR142-expressing cell" used for the screening method according to the present invention may be any of a natural cell endogenously expressing GPR142 polypeptide (for example, a normal human epidermal keratinocyte (NHEK)) and a transformed cell to which a GPR142 gene has been introduced, and a method of transforming the cell may be any of various methods known in the art. In the method according to the present invention, these cells are suitably used, but a membrane fraction of the cell containing GPR142 can also be used.

As the "GPR142 gene" used for allowing a cell to express GPR142 may be any gene encoding GPR142 derived from a mammal or a functional variant of GPR142 that retains the activity of GPR142, and the GPR142 gene is not particularly limited, but it is preferred to use a gene encoding human GPR142. Specifically, there can be used a human GPR142 gene having a base sequence encoding human GPR142 having an amino acid sequence set forth in SEQ ID NO: 1, or a human GPR142 variant that has an amino acid sequence with 90% or more, preferably 95% or more, particularly preferably 98% or more identity to the amino acid sequence set forth in SEQ ID NO: 1 and that retains the activity of GPR142 can be used. A specific example thereof is a human GPR142 gene based on the sequence set forth in GenBank accession number: NM_181790, AY288421 (FEBS Lett. 554(3), 381-388 (2003)). In particular, a human GPR142 gene having a base sequence set forth in SEQ ID NO: 2 can be suitably used.

The "GPR142-expressing cell" to which the GPR142 gene has been introduced to be used in the method according to the present invention can be obtained, for example, by transforming a host cell with an expression vector including the GPR142 gene so that the GPR142 gene is introduced into the host cell.

Any known host cell such as yeast, an insect cell, or an animal cell may be used as the host cell. For example, a CHO cell, a COS cell, a human embryonic kidney 293 (HEK293) cell, a normal human epidermal keratinocytes (NHEK cell), a human skin keratinocyte (HaCaT cell), and a Freestyle™ 293-F cell (Invitrogen) may be suitably used.

A vector containing a promoter depending on the kind of the host cell to be used is appropriately selected as the expression vector. Examples of the vector include pBR322, pBR325, pUC12, pUC13, pCXN (Gene 108: 193-199 (1991)), pUB110, pTP5, pC194, a λ phage, a retrovirus, and a baculovirus. In addition, examples of the promoter include a Trp promoter, a T7 promoter, a lac promoter, a SPO1 promoter, a penP promoter, a PHO5 promoter, a PGK promoter, a GAP promoter, a GAL promoter, an SV40 promoter, a retrovirus promoter, a metallothionein promoter, and an AG promoter.

The "GPR142-expressing cell" used for screening can be prepared by transforming a host cell with an expression vector in which the GPR142 gene is linked downstream of the promoter according to a known method. A culture medium and culture conditions for the thus obtained "GPR142-expressing cell" can be appropriately selected in the known range depending on the kind of the cell to be used.

The "activity of GPR142" in the screening method according to the present invention is a GPR142-dependent intracellular signal transduction activity, and whether a test substance is capable of promoting the "activity of GPR142" or not can be evaluated, for example, by various indicators in the signal transduction exemplified below.

For example, the signal transduction activity of GPR142 can be measured by using an adenylate cyclase activity as an indicator. The adenylate cyclase activity can be determined, for example, by measuring a concentration of (an amount of) cAMP within a cell. Specifically, for example, a GPR142-expressing cell is brought into contact with a test substance in the presence of forskolin, colforsin daropate (NKH-477), 1-methyl-3-isobutylxanthine (IBMX), or the like, or after pretreatment therewith to increase an amount of cAMP within the cell and the amount of cAMP within the cell can be measured by using cAMP EIA System (GE Healthcare), LANCE™ cAMP Detection Kit (PerkinElmer Inc.), cAMP cell-based assay (Cisbio), Cyclic AMP EIA Kit (Cayman Chemical Company), and the like. The GPR142 agonist suppresses the adenylate cyclase activity, and hence the production of cAMP is suppressed when the activity of GPR142 is promoted.

In addition, a vector in which a reporter gene such as luciferase, alkaline phosphatase, or β-galactosidase is linked via cAMP response element (CRE) is further introduced into a GPR142-expressing cell to prepare a coexpressing cell, the cell is brought into contact with a test substance in the presence of forskolin, NKH-477, IBMX, or the like or after pretreatment therewith, and the expression amount of the reporter gene can be measured.

In addition, an increase or decrease in the level of ERK1/2 phosphorylated by MAP kinase cascade caused by the stimulus of GPR142 can be measured with SureFire pERK Kit (PerkinElmer Inc.), Phospho-ERK (Cisbio), or the like, or alternatively, the level of phosphorylation of AKT in PI3K-AKT pathway caused by the stimulus of GPR142 can be measured with PathScan™ (Cell Signaling Technology Japan, K.K.), Phospho-AKT (Cisbio), Pan Specific Phospho-Akt ELISA Kit (Cayman Chemical Company), or the like.

Further, a transformed cell coexpressing a G protein chimera (Gqi) that is a chimera polypeptide of a partial polypeptide of a Gq protein having a phospholipase C-promoting activity and a partial polypeptide of a Gi protein having a coupling potency with GPR142 receptor is produced according to a conventional method, the cell is brought into contact with a test substance, and a change in the concentration of calcium ion can be measured, for example, with FLEXstation (Molecular Devices, LLC.) or the like, and the measured calcium concentration can be used as an indicator.

In the screening method according to the present invention, whether a test substance is capable of promoting the "expression of GPR142" or not can be evaluated by measuring the amount of GPR142 mRNA or protein in the GPR142-expressing cell brought into contact with the test substance, and the measurement can be conducted by extracting GPR142 mRNA or protein from the cell and measuring the amount of GPR142 mRNA or protein by a conventional method such as quantitative PCR and Western blotting.

In the screening method according to the present invention, preferably, when the activity or expression of GPR142 in a GPR142-expressing cell in the presence of a test substance is increased as compared to that in the absence of the test substance, the test substance can be determined as a substance that promotes the activity or expression of GPR142. For example, it is preferred to select, as the substance that promotes the activity of GPR142, a substance that decreases the cAMP concentration in a cell to a similar degree to the GPR142 agonist described in Examples, specifically, a substance that decreases the cAMP concentration by 10% or more, preferably by 20% or more under the conditions described in Example 9. Further, it is preferred to select, as the substance that promotes the expression of GPR142, a substance that increases the amount of GPR142 mRNA expression to a similar degree to the GPR142 agonist described in Examples, specifically, a substance that increases the amount of GPR142 mRNA expression by 1.5 times or more, preferably by 2 times or more under the conditions described in Example 7.

In addition, the method of the present invention may further include a step of determining whether a test substance selected as a substance having a GPR142 agonist activity according to the above-mentioned indicators is effective for the treatment of diseases associated with reduced FLG production or not. The therapeutic effect of a GPR142 agonist having an FLG production-promoting activity can be confirmed, for example, by methods described in Examples 10 to 14.

Thus, by screening a GPR142 agonist, a therapeutic agent for a disease associated with reduced FLG production can be screened. The GPR142 agonist according to the present invention has an FLG production-promoting action, and hence is used as a therapeutic agent for a disease, preferably a skin disease associated with reduced FLG production. Examples of the skin disease associated with reduced FLG production include atopic dermatitis, ichthyosis vulgaris, metal (nickel) allergic contact dermatitis, and asteatotic eczema (senile xerosis). However, the skin diseases are not limited to the above-mentioned diseases as long as the skin diseases are associated with reduced FLG production.

### Examples

In order to deepen the understanding of the present invention, the present invention is more specifically described by way of Examples, but it is needless to say that the present invention is not limited to Examples.

In each of Examples below, the effects of A) an N-quinolylbenzamide derivative (Compound 1), B) ivermectin (Compound 2), and C)a phenylalaninamide derivative (Compound 3) were confirmed. As a comparative compound, any one of D) Comparative Compound 1, E) Comparative Compound 2, and F) Comparative Compound 3 shown below was used.
A) Compound 1 (N-quinolylbenzamide derivative)
   N-(4-Amino-2-methyl-6-quinolyl)-2-[(4-ethylphenoxy)methyl]benzamide hydrochloride
   (N-(4-Amino-2-methylquinolin-6-yl)-2-[(4-ethylphenoxy)methyl]benzamide hydrochloride)
B) Compound 2
   Ivermectin (TOCRIS)
C) Compound 3 (phenylalaninamide derivative)
   Methyl 2-(1-(1-methyl-3-(pyridine-4-yl)-1H-pyrazol-5-ylamino)-1-oxo-3-phenylpropan-2 -ylamino)acetate dihydrochloride
D) Comparative Compound 1 (J113397) (compound described in Patent Literature 7)
   1-[(3R,4R)-1-(Cyclooctylmethyl)-3-(hydroxymethyl)-4-piperidyl]-3-ethyl -1,3-dihydro-2H-benzimidazol-2-one) hydrochloride
E) Comparative Compound 2 (scriptaid (TOCRIS))
   6-(1,3-Dioxo-1H,3H-benzo[de]isoquinolin-2-yl)hexanoic acid hydroxyamide
F) Comparative Compound 3 (3M344 (TOCRIS))
   4-Dimethylamino-N-(6-hydroxycarbamoylhexyl)benzamide

### (Example 1) Transcription-activating effect on FLG promoter

In this example, the transcription activity on FLG promoter of an N-quinolylbenzamide derivative (Compound 1) was confirmed by using neonatal normal human epidermal keratinocytes (NHEK cells). The transcription activity of the FLG promoter was measured by luciferase assay.
1) 1.2×10⁵ cells/ml of NHEK cells (KURABO INDUSTRIES LTD.) were plated on a 96-well plate at 80 µl/well (9.6×10³ cells/well), and 100 µl of a culture medium containing a medium for proliferating normal human epidermal keratinocytes (HuMedia-KG2, KURABO INDUSTRIES LTD.) supplemented with human epidermal growth factor (hEGF) (0.1 ng/ml), insulin (10 µg/ml), hydrocortisone (0.5 µg/ml), gentamycin (50 µg/ml), amphotericin B (50 ng/ml), and bovine brain pituitary extract (BPE) (0.4%, v/v) were added to the plate, and the cells were cultured under the conditions of 37°C and 5% CO₂.
2) A plasmid DNA containing 2.2-Kb FLG promoter and luciferase was prepared by introducing a promoter region from the FLG transcription initiation site ("CTTTTGGTGAACAAG" on exon 1) to about 2.2 Kb upstream thereof into the cloning site of luciferase reporter vector (pGL4 Luciferase Reporter Vectors, Promega Corporation). The sequence containing a promoter region 5' upstream of the FLG transcription initiation site is described, for example, in Ensembl Genome Browser 64: Homo sapiens-Exons-Transcript, FLG-001 (ENST00000368799), and can be confirmed by the following homepage.
   (http://www.ensembl.org/Homo_sapiens/Transcript/Exons?db=core;g=E NSG00000143631;r=1:152274651-152297679;t=ENST00000368799)
   After culture for 1 day under the conditions of the step 1), 0.1 µg of the plasmid DNA per well of the 96-well plate was added to 10 µl of OPTI-MEM (Invitrogen), and 0.3 µl of a transfection reagent (TransIT™ -LT1 Transfection Reagent, Mirus Bio LLC.) was further added to the plate to conduct the transfection into NHEK cells at room temperature.
3) After culture for additional 1 day after the step 2), the following medicament A) or B) was dissolved in dimethyl sulfoxide (DMSO), and was added to the plate at 1 µM per well. In addition, the medicament C) was added to the plate at 1 µM or 0.1 µM per well in a similar manner. Further, the system containing A) or B) plus calcium (Ca) was also confirmed. CaCl₂ (1.45 mM in terms of Ca) was used as Ca.
   A) Compound 1 (N-quinolylbenzamide derivative)
   B) Comparative Compound 1 (J113397)
   C) Comparative Compound 2 (scriptaid)

   As a control example, a solvent only (control) or CaCl₂ only (calcium) was used. It should be noted that a Ca ion is involved in the promotion of differentiation of keratinocytes in the skin in vitro.
4) 3 days after the addition of the medicament in the step 3), reagents for luciferase assay (Bright-Glo™ Luciferase Assay System, Promega Corporation) were used and the luciferase activity was measured according to the manufacturer's protocol. The cells treated with the medicament were washed twice with phosphate-buffered saline (PBS), and thereafter, 50 µl of DMEM medium (Invitrogen) were added, and then 50 µl of Bright-Glo™ reagent were added. After standing at rest for two minutes, 70 µl thereof were subjected to the measurement of the absorbance of luciferase.

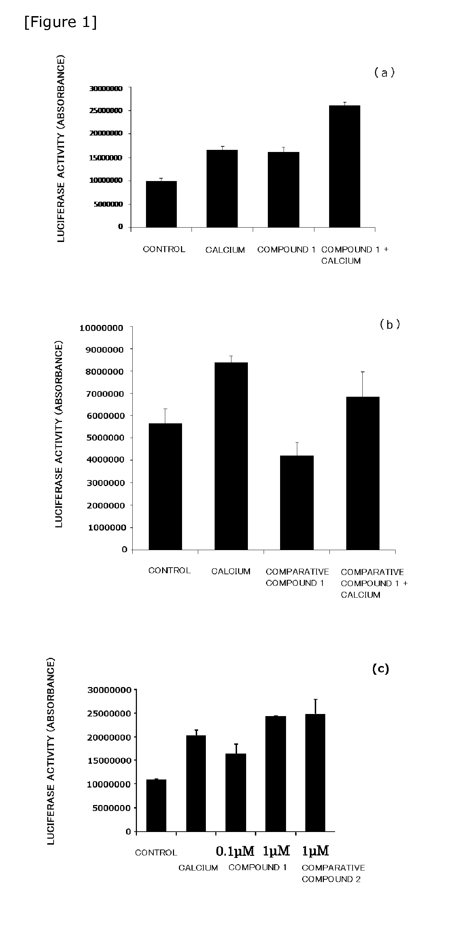

From the results shown in FIGS. 1(a), 1(b), and 1(c), it was confirmed that Compound 1 had the transcription activity on FLG promoter. Further, the transcription activity on FLG promoter was observed when Comparative Compound 2 was used. On the other hand, it was confirmed that Comparative Compound 1 (J113397) known as a nociceptin antagonist did not have the transcription activity on FLG promoter.

### (Example 2) FLG mRNA expression-increasing effect in NHEK cells

In this example, the FLG mRNA expression-increasing effect of Compound 1 or Compound 2 was confirmed by using NHEK cells.
1) 5×10⁴ cells/well of NHEK cells (Invitrogen) were plated on a 24-well plate, and then 500 µl of a culture medium containing a medium for proliferating normal human epidermal keratinocytes (HuMedia-KG2, KURABO INDUSTRIES LTD.) supplemented with hEGF (0.1 ng/ml), insulin (10 µg/ml), hydrocortisone (0.5 µg/ml), gentamycin (50 µg/ml), amphotericin B (50 ng/ml), and bovine brain pituitary extract (BPE) (0.4%, v/v) were added to the plate, and the cells were cultured under the conditions of 37°C and 5% CO₂. Next, after culture under the conditions of the following step 2) or 3), a sample was prepared.
2) After culture under the conditions of the step 1) for 1 day, the medium was exchanged for the culture medium of the step 1) to which a DMSO solution of Compound 1 or Compound 2 was added at 10 nM per well, and then culture was conducted under the conditions of 37°C and 5% CO₂. This procedure was repeated every day, and the culture was conducted for 3 days.
3) 5×10⁴ cells/well of NHEK cells (Invitrogen) were plated on a 24-well plate, and then 500 µl of a culture medium containing the culture medium of the step 1) supplemented with CaCl₂ (1.2 mM in terms of Ca) as Ca were added to the plate, and the cells were cultured under the conditions of 37°C and 5% CO₂. After culture for 1 day, the medium was exchanged for the same culture medium containing Ca, and the culture was conducted for additional 1 day (calcium priming). Thereafter, the medium was exchanged for the culture medium of the step 1) to which a DMSO solution of Compound 1 or Compound 2 was added at 10 nM per well, and the culture was further conducted for 1 day. Meanwhile, as a control example for the steps 2) and 3), a solvent only (vehicle) was added.
4) An extraction reagent (TRIzol™, Invitrogen) was used, and according to the manufacturer's protocol, total RNA was extracted from each of cells treated with the medicament (Compound 1 or Compound 2) in the steps 2) and 3). A reverse transcriptase reagent (Prime Script™, Takara) was used, and according to the manufacturer's protocol, cDNA was prepared from the total RNA. Next, real-time PCR was conducted by using the following primers and SYBR™ Premix Ex Taq (Takara) with a CFX96 real-time PCR instrument (Bio-Rad Laboratories, Inc.) according to the manufacturer's protocol, and the amount of FLG mRNA was measured. The result of the measurement is shown as a glyceraldehyde-3-phosphate dehydrogenase (GAPDH) ratio.

The sequences of the used primers are described below.
FLG Forward primer: 5'-GCTGAAGGAACTTCTGGAAAAGG-3' (SEQ ID NO: 3)
FLG Reverse primer: 5'-GTTGTGGTCTATATCCAAGTGATC-3' (SEQ ID NO: 4)
GAPDH Forward primer: 5'-GTCTCCTCTGACTTCAACAGCG-3' (SEQ ID NO: 5)
GAPDH Reverse primer: 5'-ACCACCCTGTTGCTGTAGCCAA-3' (SEQ ID NO: 6)

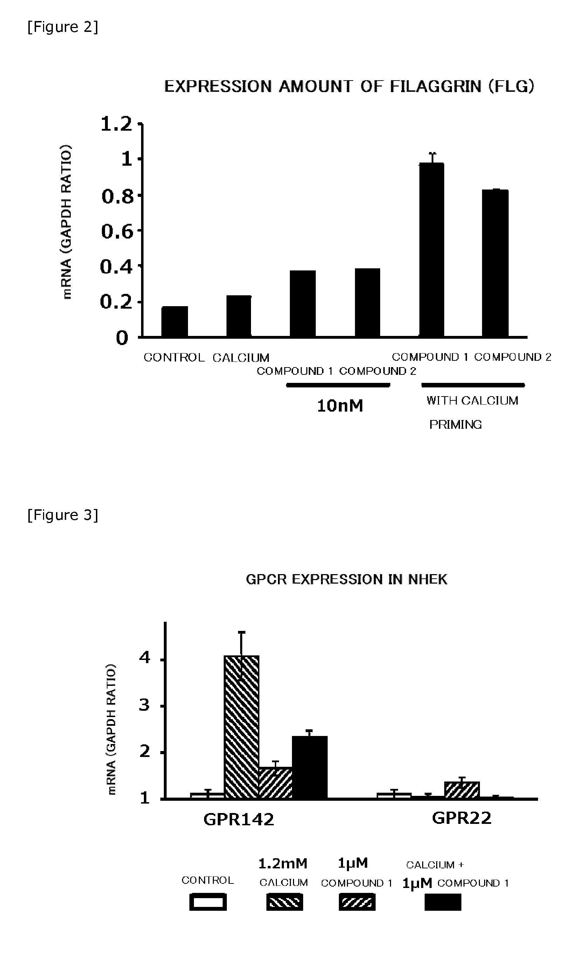

As shown in FIG. 2, it was confirmed that Compound 1 and Compound 2 increases the FLG mRNA expression in NHEK cells. It was also confirmed that the calcium priming further increased the FLG mRNA expression. When the cell-based assay of FLG production by using NHEK cells is conducted, it is preferred to conduct the calcium priming.

### (Example 3) G protein-coupled receptor(GPCR) array in NHEK cells

In this example, the expression-increasing effect of Compound 1 on a GPCR in NHEK cells was confirmed by using a GPCR array.
1) 5×10⁴ cells/well of NHEK cells (Invitrogen) were plated on a 24-well plate, and then 500 µl of a culture medium containing a medium for proliferating normal human epidermal keratinocytes (HuMedia-KG2, KURABO INDUSTRIES LTD.) supplemented with hEGF (0.1 ng/ml), insulin (10 µg/ml), hydrocortisone (0.5 µg/ml), gentamycin (50 µg/ml), amphotericin B (50 ng/ml), and bovine brain pituitary extract (BPE) (0.4%, v/v) were added to the plate, and the cells were cultured under the conditions of 37°C and 5% CO₂ for from 3 to 4 days until the 80 to 85% confluent state was achieved.
2) Next, a DMSO solution of Compound 1 was added to the medium at 1 µM per well, and culture was further conducted for 3 days without changing the medium. In addition, regarding the system to which only CaCl₂ (1.5 mM in terms of Ca) as Ca was added and the system to which both Compound 1 and CaCl₂ (1.5 mM in terms of Ca) as Ca were added, the culture was conducted in a similar manner for 3 days. As a control example, a solvent only (vehicle) was added.
3) An extraction reagent (TRIzol™, Invitrogen) was used, and according to the manufacturer's protocol, total RNA was extracted from each of cells treated in the step 2). A reverse transcriptase reagent (Prime Script™, Takara) was used, and according to the manufacturer's protocol, cDNA was prepared from the total RNA. Next, a TaqMan™ human GPCR array (AB 4365295, Applied Biosystems) was used, and the analysis was conducted by an ABI PRISM™ 7900HT gene analyzer (Applied Biosystems). The result of measurement was shown as the comparison of the cycle number (ΔCt value) to 18S ribosomal RNA as a control (comparative Ct method).

Table 1 shows the result of GPCR showing that the treatment with Compound 1 and/or calcium markedly increased the expression as compared to the control. As shown in Table 1, it was found that calcium induced GPR142 mRNA expression in NHEK cells. In addition, it was confirmed that GPR142 mRNA and GPR22 mRNA expression was induced in the system in which Compound 1 was used and the system in which both Compound 1 and calcium were used.

**[Table 1]**

| G protein-coupled receptor (GPCR) array in NHEK cells | | | | |
|---|---|---|---|---|
| | Culture conditions | | | |
| | | | | |
| | Control | Calcium | Compound 1 | Calcium+Compound 1 |
| GPR142 | 29.2 | -4.9 | -4.9 | 0.28 |
| GPR22 | 29.2 | 29.2 | 6.7 | 17.7 |

### (Example 4) Confirmation of expression of GPR142 and GPR22 in NHEK cells

In this example, the expression-inducing effects of Compound 1 on GPR142 mRNA and GPR22 mRNA in NHEK cells were confirmed.
1) 5×10⁴ cells/well of NHEK cells (Invitrogen) were plated on a 24-well plate. Then, 500 µl of a culture medium containing a medium for proliferating normal human epidermal keratinocytes (HuMedia-KG2, KURABO INDUSTRIES LTD.) supplemented with hEGF (0.1 ng/ml), insulin (10 µg/ml), hydrocortisone (0.5 µg/ml), gentamycin (50 µg/ml), amphotericin B (50 ng/ml), and bovine brain pituitary extract (BPE) (0.4%, v/v) were added to the plate, and the cells were cultured under the conditions of 37°C and 5% CO₂.
2) After culture under the conditions of the step 1) for 1 day, the medium was exchanged for the culture medium of the step 1) to which a DMSO solution of Compound 1 was added at 1 µM per well every day and the culture was conducted for 3 days. Further, regarding the system in which CaCl₂ (1.2 mM in terms of Ca) was used as Ca instead of Compound 1 and the system in which both Ca (1.2 mM in terms of Ca) and Compound 1 (1 µM per well) were used, culture was conducted in a similar manner for 3 days. As a control, a solvent only (vehicle) was added.
3) An extraction reagent (TRIzol™, Invitrogen) was used, and according to the manufacturer's protocol, total RNA was extracted from each of cells treated with the medicament. A reverse transcriptase reagent (Prime Script™, Takara) was used, and according to the manufacturer's protocol, cDNA was prepared from the total RNA. Next, real-time PCR was conducted by using the following primers and SYBR™ Premix Ex Taq (Takara) with a CFX96 real-time PCR instrument according to the manufacturer's protocol, and the amounts of GPR142 mRNA and GPR22 mRNA expression were measured. The result of measurement is shown as a GAPDH ratio.

The sequences of the used primers are described below.
GPR142 Forward primer: 5'-CTTGTCTCAGCCATTCCAGGTG-3' (SEQ ID NO: 7)
GPR142 Reverse primer: 5'-GATCCTTACGCAGACACTGAGC-3' (SEQ ID NO: 8)
GPR22 Forward primer: 5'-CCACACAACATGAGGCTACAGAC-3' (SEQ ID NO: 9)
GPR22 Reverse primer: 5'-CTTTCTCGTCGTTCACGGTGTC-3' (SEQ ID NO: 10)
GAPDH Forward primer: 5'-GTCTCCTCTGACTTCAACAGCG-3' (SEQ ID NO: 5)
GAPDH Reverse primer: 5'-ACCACCCTGTTGCTGTAGCCAA-3' (SEQ ID NO: 6)

As shown in FIG. 3, it was confirmed that GPR142 mRNA expression was induced in NHEK cells in the system containing calcium, the system containing Compound 1, and the system containing both Compound 1 and calcium as compared to the control. In addition, it was confirmed that Compound 1 induced GPR22 mRNA expression. It was confirmed that these experimental results were consistent with the results of the GPCR array (expression of GPR142 and GPR22) of Example 3 described above.

### (Example 5) Blocking effect by GPR142 RNAi

In this example, it was confirmed that the expression-inducing effects of Compound 1 on GPR142 mRNA and FLG mRNA in NHEK cells were blocked by GPR142 RNAi.
1) 5×10⁴ cells/well of NHEK cells (Invitrogen) were plated on a 24-well plate. Then, 500 µl of a culture medium containing a medium for proliferating normal human epidermal keratinocytes (HuMedia-KG2, KURABO INDUSTRIES LTD.) supplemented with hEGF (0.1 ng/ml), insulin (10 µg/ml), hydrocortisone (0.5 µg/ml), gentamycin (50 µg/ml), amphotericin B (50 ng/ml), and bovine brain pituitary extract (BPE) (0.4%, v/v) were added to the plate, and the cells were cultured under the conditions of 37°C and 5% CO₂.
2) After culture under the conditions of the step 1) for 1 day, the medium was exchanged for the culture medium of the step 1) to which CaCl₂ (1.2 mM in terms of Ca) was further added as Ca, and culture was conducted under the conditions of 37°C and 5% CO₂. After culture for 1 day, the medium was exchanged for the same culture medium containing Ca, and culture was further conducted for 1 day.
3) The NHEK cells of the step 2) were transfected with 50 nM of siRNA per well of the 24-well plate by using a transfection reagent, Lipofectamine RNA Max (Invitrogen) according to the manufacturer's protocol.
   The sequences of the used three kinds of GPR142 siRNAs and the sequences of the used control siRNAs (all the siRNAs are manufactured by Invitrogen) are described below.
   GPR142-1 (#HSS139493):
      Sense Sequence: GGCGUCAUCCCUGUCAUCUACUACA; (SEQ ID NO: 11)
      Antisense Sequence: UGUAGUAGAUGACAGGGAUG; (SEQ ID NO: 12)
   GPR142-2 (#HSS139494):
      Sense Sequence: CCAACAUCCUGGAGUUUGCUGCCAA; (SEQ ID NO: 13)
      Antisense Sequence: UUGGCAGCAAACUCCAGGAUGU; (SEQ ID NO: 14)
   GPR142-3 (#HSS179846):
      Sense Sequence: GCUGUCCUGAGUGCUGCCCUGUUGA; (SEQ ID NO: 15)
      Antisense Sequence: UCAACAGGGCAGCACUCAGGAC; (SEQ ID NO: 16)
   Control-1 (LOW GC, #129350-200):
      Sense Sequence: GGAUGUCAACUUGAGCAACUUAUUU; (SEQ ID NO: 17)
      Antisense Sequence: AAAUAAGUUGCUCAAGUUGACAUCC; (SEQ ID NO: 18)
   Control-2 (MED GC, #129350-300);
      Sense Sequence: GGUAGGUGAGUGUACAGACGCAAUA; (SEQ ID NO: 19)
      Antisense Sequence: UAUUGCGUCUGUACACUCACCUACC; (SEQ ID NO: 20)
   Control-3 (HIGH GC, #129350-400):
      Sense Sequence: GGCGGCUCGUGCAACACAGGCGCUU; (SEQ ID NO: 21)
      Anti Sense Sequence: AAGCGCCUGUGUUGCACGAGCCGCC; (SEQ ID NO: 22)
4) After culture for 1 day after the transfection with siRNA in the step 3), the medium was exchanged for the culture medium of the step 1) to which a DMSO solution of Compound 1 was added at 10 nM per well, and culture was further conducted for 1 day. As a control, a solvent only (vehicle) was added.
5) An extraction reagent (TRIzol™, Invitrogen) was used, and according to the manufacturer's protocol, total RNA was extracted from each of cells treated with the medicament. A reverse transcriptase reagent (Prime Script™, Takara) was used, and according to the manufacturer's protocol, cDNA was prepared from the total RNA. Next, real-time PCR was conducted by using the following primers and SYBR™ Premix Ex Taq (Takara) with a CFX96 real-time PCR instrument according to the manufacturer's protocol, and the amounts of GPR142 mRNA and FLG mRNA expression were measured. The result of measurement is shown as a GAPDH ratio.

The sequences of the used primers are described below.
GPR142 Forward primer: 5'-CTTGTCTCAGCCATTCCAGGTG-3' (SEQ ID NO: 7)
GPR142 Reverse primer: 5'-GATCCTTACGCAGACACTGAGC-3' (SEQ ID NO: 8)
FLG Forward primer: 5'-GCTGAAGGAACTTCTGGAAAAGG-3' (SEQ ID NO: 3)
FLG Reverse primer: 5'-GTTGTGGTCTATATCCAAGTGATC-3' (SEQ ID NO: 4)
GAPDH Forward primer: 5'-GTCTCCTCTGACTTCAACAGCG-3' (SEQ ID NO: 5)
GAPDH Reverse primer: 5'-ACCACCCTGTTGCTGTAGCCAA-3' (SEQ ID NO: 6)

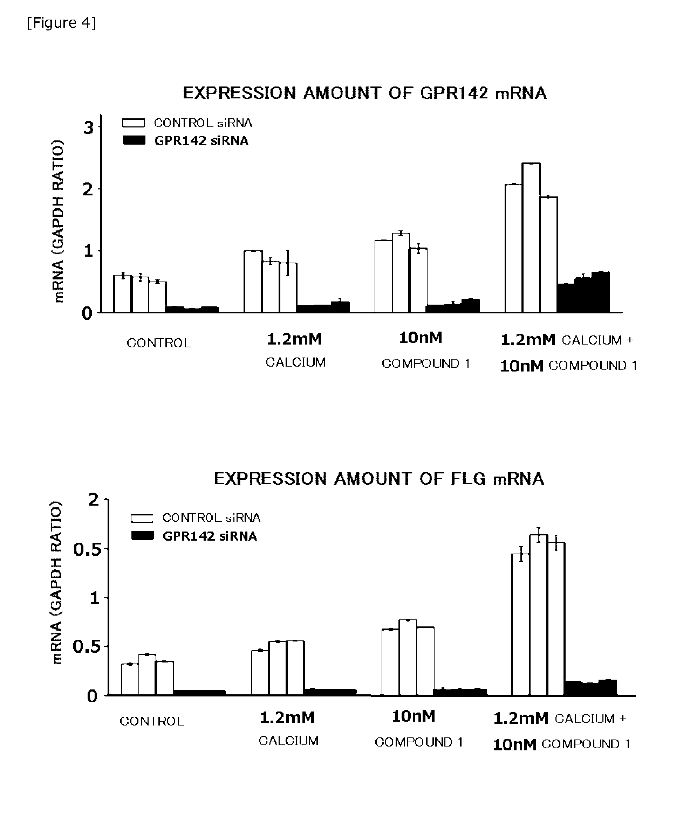

As shown in FIGS. 4, it was confirmed that FLG mRNA expression shown in Example 4 was mediated by GPR142 because GPR142 mRNA and FLG mRNA expression was suppressed by GPR142 siRNA in the system containing calcium, the system containing Compound 1, and the system containing both calcium and Compound 1.

### (Example 6) Blocking effect by GPR142 RNAi

In this example, it was confirmed that the expression-inducing effects of Compound 2 or 3 on GPR142 mRNA and FLG mRNA in NHEK cells were blocked by GPR142 RNAi.

NHEK cells were transfected in the same manner as in Example 5 except that, in the steps 1) to 3) of Example 5, two kinds of GPR142 siRNAs and two kinds of control siRNAs, i.e., GPR142-1 (#HSS139493) and GPR142-3 (#HSS179846) and Control-1 (LOW GC, #129350-200), and Control-3 (HIGH GC, #129350-400) were used as GPR142 siRNAs and control siRNAs, respectively. After culture for 1 day after the transfection was conducted in the same manner as in the step 4) of Example 5, the medium was exchanged for the culture medium to which each of a DMSO solution of Compound 1, a DMSO solution of Compound 2, or a methanol solution of Compound 3 was added at 10 nM per well, and culture was further conducted for 1 day. As a control, a solvent only (vehicle) was added. Regarding each of cells treated with the medicament, real-time PCR was conducted to measure the amounts of GPR142 mRNA and FLG mRNA in the same manner as in the step 5) of Example 5. The results of the measurement are shown as a GAPDH ratio.

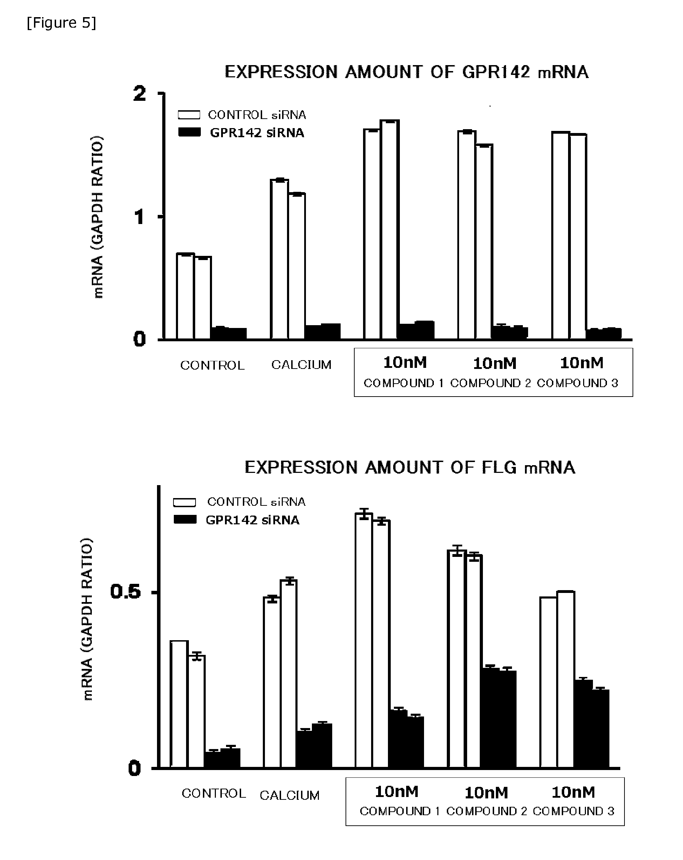

As shown in FIGS. 5, it was confirmed that the induction of FLG mRNA expression by Compound 2 and Compound 3 was mediated by GPR142 as in Compound 1.

### (Reference Example 1) Blocking effect by GPR22 RNAi

In this reference example, it was confirmed that the FLG mRNA expression-inducing effect of Compound 1 in NHEK cells was not suppressed by GPR22 RNAi-mediated blocking.
1) 5×10⁴ cells/well of NHEK cells (Invitrogen) were plated on a 24-well plate. Then, 500 µl of a culture medium containing a medium for proliferating normal human epidermal keratinocytes (HuMedia-KG2, KURABO INDUSTRIES LTD.) supplemented with hEGF (0.1 ng/ml), insulin (10 µg/ml), hydrocortisone (0.5 µg/ml), gentamycin (50 µg/ml), amphotericin B (50 ng/ml), and bovine brain pituitary extract (BPE) (0.4%, v/v) were added to the plate, and the cells were cultured under the conditions of 37°C and 5% CO₂.
2) 1 day after the step 1), the medium was exchanged for the medium supplemented with calcium every day, and culture was conducted for 2 days (priming).
3) The NHEK cells of the step 2) were transfected with 10 nM of siRNA per well of the 24-well plate by using a transfection reagent, Lipofectamine RNA Max (Invitrogen) according to the manufacturer's protocol.
   The sequences of the used siRNAs are described below.
   GPR22-1 (#HSS104365):
      Sense Sequence: UAGUCAUGGCUUAUGGAACAACUAU (SEQ ID NO: 23)
      Anti Sense Sequence: AUAGUUGUUCCAUAAGCCAUGACUA (SEQ ID NO: 24)
   GPR22-2 (#HSS104367):
      Sense Sequence: GAGUUGUUUCUAUAGUAGAAGCUGA (SEQ ID NO: 25)
      Anti Sense Sequence: UCAGCUUCUACUAUAGAAACAACUC (SEQ ID NO: 26)
   GPR22-3 (#HSS178711):
      Sense Sequence: GGCAGAGCUGUAAUGUUAAUGAUAU (SEQ ID NO: 27)
      Anti Sense Sequence: AUAUCAUUAACAUUACAGCUCUGCC (SEQ ID NO: 28)
   Control-1 (Low GC, #129350-200):
      Sense Sequence: GGAUGUCAACUUGAGCAACUUAUUU (SEQ ID NO: 17)
      Anti Sense Sequence: AAAUAAGUUGCUCAAGUUGACAUCC (SEQ ID NO: 18)
   Control-2 (MED GC, #129350-300):
      Sense Sequence: GGUAGGUGAGUGUACAGACGCAAUA (SEQ ID NO: 19)
      Anti Sense Sequence: UAUUGCGUCUGUACACUCACCUACC (SEQ ID NO: 20)
   Control-3 (HIGH GC, #129350-400):
      Sense Sequence: GGCGGCUCGUGCAACACAGGCGCUU (SEQ ID NO: 21)
      Anti Sense Sequence: AAGCGCCUGUGUUGCACGAGCCGCC (SEQ ID NO: 22)
4) After culture for 1 day after the step 3), the medium was exchanged for a medium to which Compound 1 dissolved in DMSO was added at 10 nM per well on the 24-well plate, and culture was conducted for 1 day. As a control example, a solvent only (vehicle) was added.
5) An extraction reagent (TRIzol™, Invitrogen) was used, and according to the manufacturer's protocol, total RNA was extracted from each of cells treated with the medicament. A reverse transcriptase reagent (Prime Script™, Takara) was used, and according to the manufacturer's protocol, cDNA was prepared from the total RNA. Real-time PCR was conducted by using SYBR™ Premix Ex Taq (Takara) with a CFX96 real-time PCR instrument according to the manufacturer's protocol, and the amounts of GPR22 mRNA and FLG mRNA were measured. The results of measurement are shown as a GAPDH ratio.

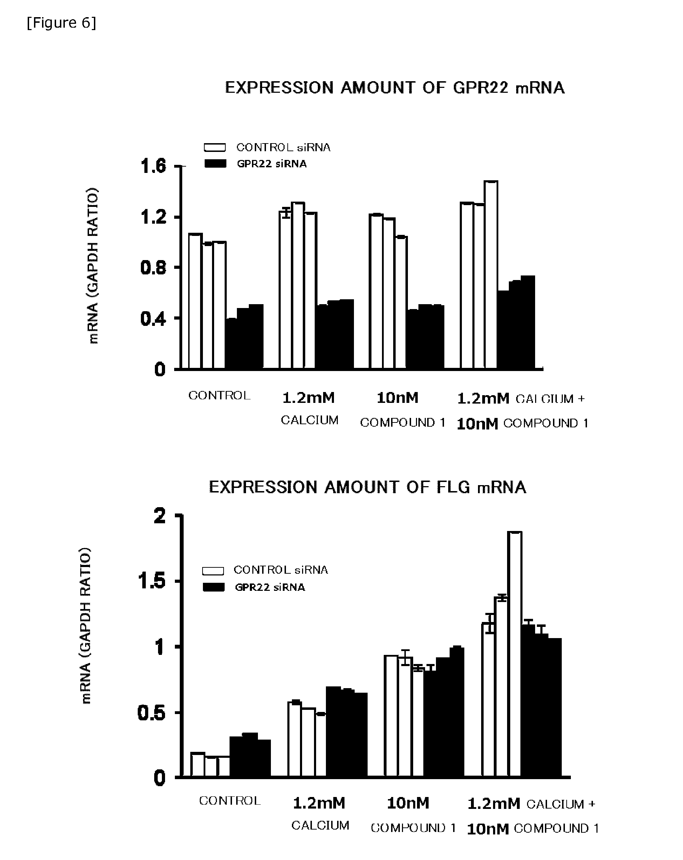

As shown in FIGS. 6, in the system containing calcium, the system containing Compound 1, and the system containing both calcium and Compound 1, GPR22 mRNA expression was suppressed by GPR22 siRNA as compared to the control, but FLG mRNA expression was not suppressed. It was confirmed, from the results of Examples 5 and 6, that FLG mRNA expression induced by Compound 1 was mediated by GPR142.

### (Example 7) FLG mRNA-producing effect of Compound 1 in GPR142-overexpressing NHEK cells

In this example, the FLG mRNA expression-inducing effect of Compound 1 in GPR142-overexpressing NHEK cells was confirmed.
1) 5×10⁴ cells/well of NHEK cells (Invitrogen) were plated on a 24-well plate. Then, a culture medium containing a medium for proliferating normal human epidermal keratinocytes (HuMedia-KG2, KURABO INDUSTRIES LTD.) supplemented with hEGF (0.1 ng/ml), insulin (10 µg/ml), hydrocortisone (0.5 µg/ml), gentamycin (50 µg/ml), amphotericin B (50 ng/ml), and bovine brain pituitary extract (BPE) (0.4%, v/v) was added to the plate, and the cells were cultured under the conditions of 37°C and 5% CO₂.
2) A GPR142pCXN mammalian expression vector was prepared by incorporating a human GPR142 gene coding region containing the base sequence set forth in SEQ ID NO: 2 into the EcoRI restriction site of the cloning site in a mammalian expression vector pCXN (Gene 108: 193-199 (1991)).
3) After culture for 1 day under the conditions of the step 1), the NHEK cells were transfected with 0.5 µg of the GPR142-mammalian expression vector pCXN per well by using a transfection reagent Fugene™ HD (Promega Corporation) according to the manufacturer's protocol. As a control, only the mammalian expression vector pCXN was transfected.
4) After culture for 1 day after the transfection as described above, the medium was exchanged for the culture medium to which a DMSO solution of Compound 1 was added at 10 nM per well, and culture was further conducted for 1 day. As a control, a solvent only (vehicle) was added.
5) An extraction reagent (TRIzol™, Invitrogen) was used, and according to the manufacturer's protocol, total RNA was extracted from each of cells treated with the medicament. A reverse transcriptase reagent (Prime Script™, Takara) was used, and according to the manufacturer's protocol, cDNA was prepared from the total RNA. Real-time PCR was conducted by using the same primers as those for GPR142, FLG, and GAPDH used in Example 4 and SYBR™ Premix Ex Taq (Takara) with a CFX96 real-time PCR instrument according to the manufacturer's protocol, and the amounts of GPR142 mRNA and FLG mRNA were measured. The results of the measurement are shown as a GAPDH ratio.

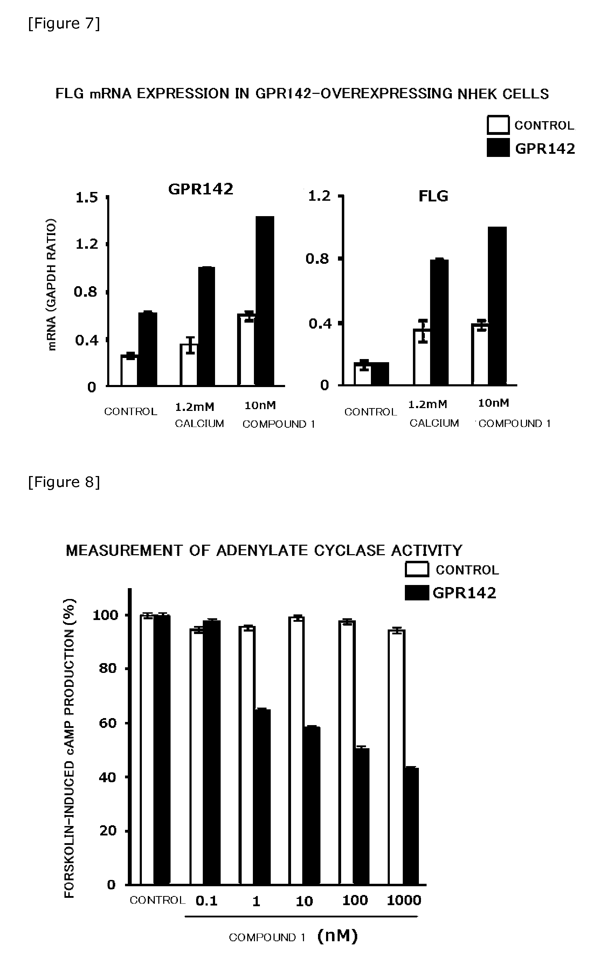

As shown in FIGS. 7, it was confirmed that Compound 1 increased FLG mRNA expression in GPR142-overexpressing NHEK cells. From these facts, it was confirmed that GPR142 was involved in FLG expression and that Compound 1 was involved in FLG expression via GPR142. Further, it was confirmed that Compound 1 induced GPR142 in NHEK cells (the expression amount of GPR142 mRNA was increased by about 2.2 times as compared to the control).

### (Example 8) Measurement of action on adenylate cyclase activity

In this example, the cAMP production-suppressing effect of Compound 1 was confirmed.
1) 6×10⁴ cells/well of HEK293T cells were plated on a collagen-coated 24-well plate, a culture medium containing DMEM medium (Invitrogen) supplemented with penicillin (50 µg/ml), streptomycin (50 µg/ml), and fetal bovine serum (FBS) (10%, v/v) was added to the plate, and the cells were cultured under the conditions of 37°C and 5% CO₂.
2) A GPR142PCXN mammalian expression vector was prepared in the same manner as in the step 2) of Example 7.
3) After culture for 1 day under the conditions of the step 1), HEK293T cells were transfected with 0.2 µg of the GPR142PCXN mammalian expression vector per well by using a transfection reagent Fugene™ HD (Promega Corporation) according to the manufacturer's protocol. As a control, cells transfected only with the PCXN mammalian expression vector were used.
4) After culture for 1 day after the transfection described above, 1 mM of isobutylmethylxanthine (IBMX), which was a cAMP phosphodiesterase inhibitory substance, was added to HEK293T cells, and thereafter, the cells were cultured for 10 minutes at 37°C.
5) Next, the medium was exchanged for the culture medium to which 0.1 nM to 1000 nM of a DMSO solution of Compound 1, 10 µM of forskolin, and 1 mM of IBMX per well were added, and the cells were further cultured for 20 minutes at 37°C. As a control, a solvent (vehicle), 10 µM of forskolin, and 1 mM of IBMX were added.
6) The cells cultured in the step 5) were lysed by 0.1 mol/L of HCl. The cAMP concentration within the cells was measured by using Cyclic AMP EIA Kit (Cayman Chemical Company) according to the manufacturer's protocol. The result is shown as the ratio (%) of the cAMP concentration in the cells treated with Compound 1 with respect to the cAMP concentration in the control example defined as 100%.

As shown in FIG. 8, it was confirmed that Compound 1 decreased cAMP production via GPR142 dose-dependently.

### (Example 9) Measurement of action on adenylate cyclase activity

In this example, the cAMP production-suppressing effect of Compound 2 or 3 was confirmed.

HEK293T cells were cultured in the same manner as in the steps 1) to 4) of Example 8. Next, the cells were cultured in the same manner as in the step 5) of Example 8 except that, in the step 5) of Example 8, each of a methanol solution of Compound 3, a DMSO solution of Compound 2, and a DMSO solution of Comparative Compound 2 was used at 1,000 nM per well instead of Compound 1. The cAMP concentration in the cultured cells was measured in the same manner as in the step 6) of Example 8.

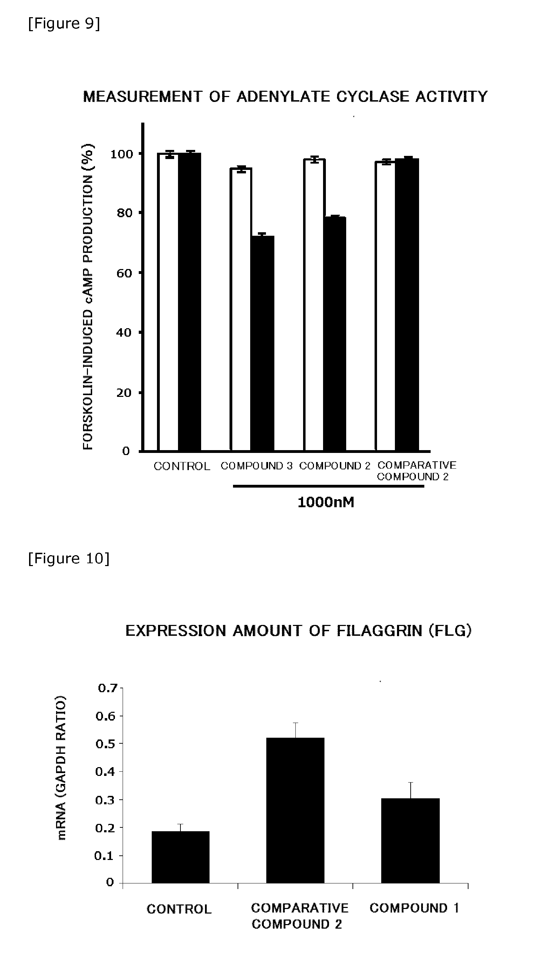

As shown in FIG. 9, it was confirmed that Compound 3 and Compound 2 decreased cAMP production via GPR142 (decreases by about 24% and about 20%, respectively). On the other hand, no cAMP production-suppressing action was observed in the stimulation with Comparative Compound 2.

### (Example 10) Expression-increasing effect on FLG mRNA in three-dimensional cultured skin

In this example, the expression-increasing effect of Compound 1 on FLG mRNA was examined by using three-dimensional cultured skin. FLG mRNA expression was measured by a real-time PCR method.
1) A commercially available human skin three-dimensional model (TEST SKIN™ LSE-013, TOYOBO CO., LTD.) was cultured by using TEST SKIN LSE ASSAY MEDIUM (TOYOBO CO., LTD.) according to the manufacturer's protocol.
2) After culture for 2 days, the following medicament A) or C) was dissolved in DMSO, and was added to the medium at 1 µM to treat the cells with the medicament.
   A) Compound 1 (N-quinolylbenzamide derivative)
   C) Comparative Compound 2 (scriptaid)

   As a control example, a solvent only (vehicle) was added.
3) After further culture for 2 days (4 days after the start of culture), the medium was exchanged for the medium to which the medicament was added. 3 days after further culture (7 days after the start of culture), the three-dimensional cultured skin was recovered, frozen with liquid nitrogen, and then stored at -30°C.
4) Total RNA was extracted from each of the cells treated with the medicaments by using an RNA extraction reagent (RNeasy™, QIAGEN N.V.) according to the manufacturer's protocol. cDNA was prepared from the total RNA by using a reverse transcriptase reagent (Prime Script™, Takara) according to the manufacturer's protocol. Real-time PCR was performed by using Light Cycler 480 SYBR Green I Master (Roche) according to the manufacturer's protocol to measure the amount of FLG mRNA. The result of measurement is shown as a GAPDH ratio.

As shown in FIG. 10, it was confirmed that Compound 1 and Comparative Compound 2 increased FLG mRNA expression in the human skin three-dimensional model.

### (Example 11) Expression-increasing effect on FLG monomer in three-dimensional cultured skin

In this example, the expression-increasing effect of Compound 1 on FLG monomer was examined by using a three-dimensional cultured skin. The expression of the FLG monomer was confirmed by Western blotting.

In this example, the same procedures as those in the steps 1) to 3) of Example 10 were conducted. It should be noted that as a medicament, D) Comparative Compound 3 (M344) was used, as well as A) Compound 1 and C) Comparative Compound 2 of Example 10.
4) The three-dimensional cultured skin obtained in the same manner as in the steps 1) to 3) of Example 10 was recovered, and was cut into pieces with scissors. Then, 100 µl of Lysis buffer (0.1 M Tris-HCl (pH 9.0), 6 M Urea, 1% 2-mercapto-ethanol, 1% SDS, 1 mM EDTA, and Protease inhibitor cocktail) were added and the cells were homogenized with an electrically powered pestle. The homogenized cells were centrifuged at 15,000 rpm at 4°C for 20 minutes, and the supernatant was recovered to be used as a sample for measurement. The total protein in the sample was quantified by using a protein assay reagent (Bio-Rad Laboratories, Inc.) according to the manufacturer's protocol. The protein was adjusted so that each sample contained 15 µg protein/lane, and the samples were subjected to electrophoresis by SDS-PAGE (Invitrogen, NuPAE Novex Bis-Tris mini Gel 4 to 12%) under the conditions of 100 V and 2 hours. As a marker, Precision Plus Protein Standards (Bio-Rad Laboratories, Inc.) was used. The proteins separated by the electrophoresis were transferred to a PVDF membrane (blotting), and the expression of FLG monomer was confirmed by using an anti-FLG antibody (Filaggrin AKH1, Santa Crus). At that time, Anti-mouse IgG-HRP (GE Healthcare) was used as a secondary antibody, and Amersham ECL Plus Western Blotting Detection Reagents (GE Healthcare) was used as a color developer.

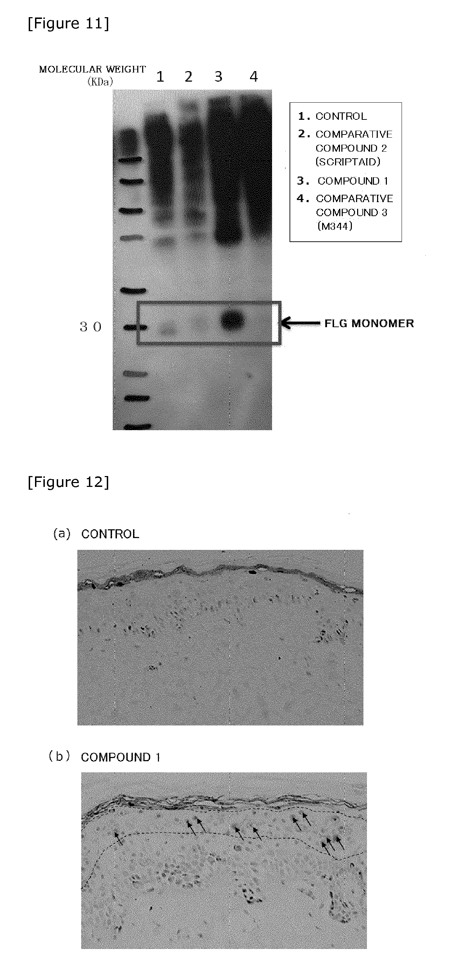

It was found that Compound 1 caused the production of a large amount of pro-FLG from the result shown in FIG. 11. In addition, in the case of Compound 1, the clear production of FLG monomer was confirmed. On the other hand, in the cases of Comparative Compound 2 and Comparative Compound 3, the clear production of FLG monomer was not observed.

### (Example 12) Expression-increasing effect on FLG monomer in cultured human normal skin

In this example, the expression-increasing effect of Compound 1 (N-quinolylbenzamide derivative) on FLG monomer was examined by using human normal skin. The expression of the FLG monomer was confirmed by an immunohistostaining method using an anti-human FLG antibody. The medium used was a medium for proliferating normal human epidermal keratinocytes (HuMedia-KG2, KURABO INDUSTRIES LTD.). It should be noted that, in this example, surplus portions of normal skin derived from extirpation of portions of a skin disease were used under the agreement by the donor through Ethics Committee of Kyoto University.
1) Human normal skin (with a size of 5x5x5 mm) was put on a transwell of a 24-well transwell plate, 500 µl of the medium, to which Compound 1 (1 µM) or a control (vehicle (DMSO)) was added, were added to the lower part, and the skin was cultured. After culture for 2 days, the medium was exchanged for the same medium.
2) After 4 days, the cultured skin was collected, and was soaked in 10% formalin overnight, and thereafter, a paraffin section having a thickness of 6 mm was prepared according to a conventional method. After deparaffinization was conducted according to a conventional method, the expression state of FLG protein was confirmed by immunohistostaining by using an anti-human FLG antibody (Anti-Human Filaggrin Antibody sc-66192, Santa Cruz Biotechnology, Inc.) as a primary antibody and a biotinylated anti-mouse IgG antibody (eBioscience, Inc.) as a secondary antibody and by using diaminobenzidine (DAB) as a color developer.

In FIG. 12(b), markedly stained portions, in an area surrounded by dotted lines are indicated by arrows. From the results shown in (a) and (b), it was confirmed that the development of the stratum corneum of (b) to which Compound 1 was added was greater than that of the control (a) and that FLG protein within the skin of (b) was markedly increased as compared to that of (a).

### (Example 13) Expression-increasing effect on FLG in model animals

In this example, the expression-increasing effect of Compound 1 (N-quinolylbenzamide derivative) on FLG monomer was confirmed by using FLG gene homozygous or heterozygous mutant mouse (Jackson Laboratory, flaky tail mice). The expression of the FLG protein was confirmed by Western blotting.
1) Compound 1 (1 µM) dissolved in ultrapure water (milliQ, EMD Millipore) was injected intradermally to left and right ears of an FLG homozygous or heterozygous mutant mouse (10 µl/ear). As a control, ultrapure water (milliQ, EMD Millipore) was injected intradermally in the same manner.
2) After 3 days, Compound 1 or ultrapure water was further injected intradermally in the same manner.
3) After 8 days, ears of each mouse were cut under anesthesia, the skin of the cut ear was peeled off by pinching cartilage, and the skin on the dermal side was soaked in PBS containing 20 mM EDTA at 37°C for 30 minutes. Next, the epidermis of the ear was peeled off and 50 ml of Lysis buffer (0.1 M Tris-HCl (pH 9.0), 6 M Urea, 1% 2-mercapto-ethanol, 1% SDS, 1 mM EDTA, and Protease inhibitor cocktail) were added to the epidermis, and the epidermis was homogenized with an electrically powered pestle in the same manner as in the step 4) of Example 11 to prepare a sample. The sample was subjected to electrophoresis, and then to western blotting to confirm the expression of FLG monomer. 1 µg/ml of an anti-mouse FLG antibody (Anti-Mouse Filaggrin Antibody, Covance Inc.) was used as a primary antibody, Anti-Rabbit IgG-HRP (GE Healthcare) was used as a secondary antibody, and Amersham ECL Plus Western Blotting Detection Reagents (GE Healthcare) was used as a color developer.

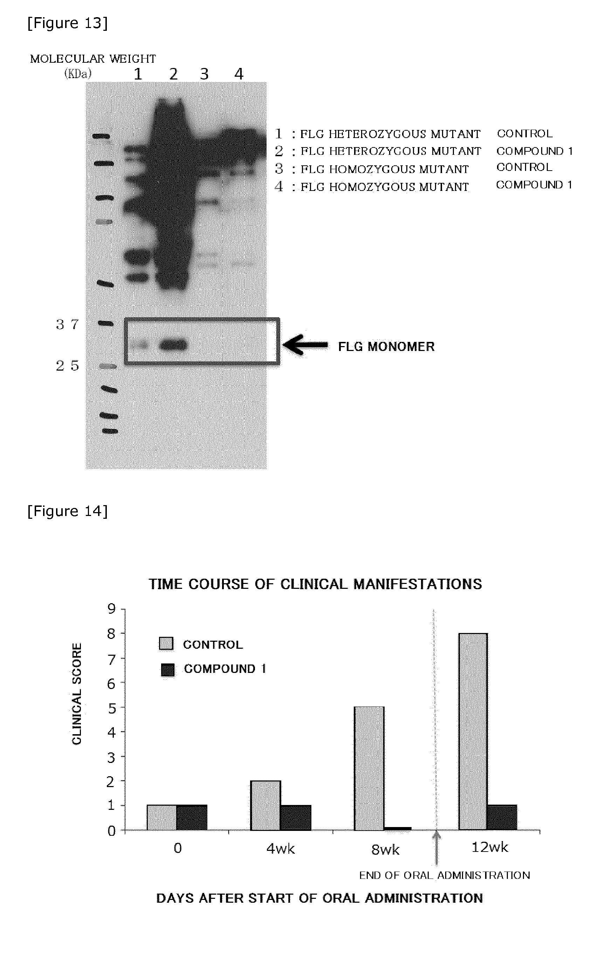

From the result shown in FIG. 13, the production of the FLG monomer caused by Compound 1 was confirmed in the FLG heterozygous mutant mouse. On the other hand, the production of the FLG monomer was not observed in the FLG homozygous mutant mouse. It is supposed from these results that Compound 1 induced the transcription of FLG gene, which led to the expression of pro-FLG, and then, the FLG monomer was produced through a series of reactions including dephosphorylation and hydrolysis.

### (Example 14) Dermatitis-alleviating effect in atopic dermatitis mouse model (NC/Nga)

Experiments were conducted with raising 6- to 10-week-old atopic dermatitis-developing NC/Nga mice with mites (Japan SLC, Inc.) under a normal environment. Namely, the dermatitis-alleviating effect was measured by the oral administration of 500 µl of a solution obtained by dissolving Compound 1 at 0.75 mg/ml in a methyl cellulose solution (0.5 W/V% Methyl Cellulose 400 Solution, Wako, 133-14255) to an NC/Nga mouse developing dermatitis majorly in the ear auricles and the head (0.375 mg of Compound 1 was administered to the mouse per time) every other day. As a control, 500 µl of the methylcellulose solution were administered orally every other day.

### (i) Observation of clinical manifestations of dermatitis

With regard to clinical manifestations, the evaluation was conducted on a four-point scale of none (0), weak (1), moderate (2), and severe (3) by visual inspection for four items of erythema, edema, erosion, and squama. The evaluated values of the four items were summed to give a score before the start of oral administration (0 wk), 4 weeks after the oral administration (4 wk), and 8 weeks after the oral administration (8 wk), and then the clinical manifestations of a mouse model were measured as a clinical score. Further, the oral administration was ended 8 weeks after the start of oral administration, and thereafter, the mouse was continued to be kept under the same conditions. 4 weeks after the end of oral administration (12 wk after the start of oral administration), clinical manifestations were scored in the same manner. The results are shown in FIG. 14.

As apparent from FIG. 14, the symptoms of dermatitis were alleviated 8 weeks after the start of oral administration of Compound 1. In addition, 4 weeks after the end of oral administration, the symptoms of dermatitis were suppressed as compared to the control.

### (ii) Measurement of amount of water transpiration (transepidermal water loss: TEWL)

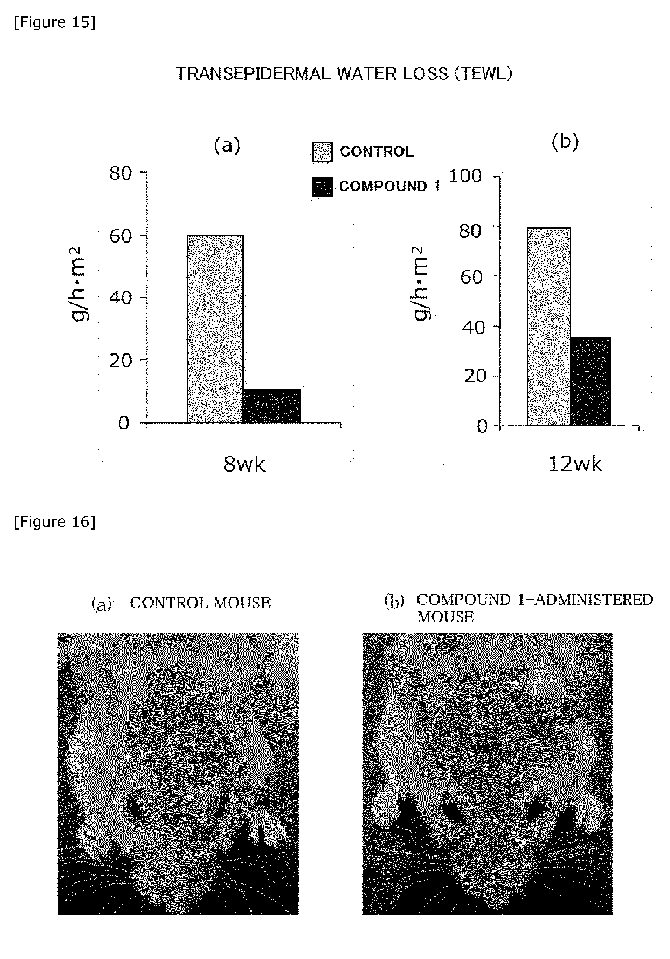

A TEWL was measured 8 and 12 weeks after the start of oral administration in the above-mentioned section (i) using a tape type closed chamber system water transpiration measuring instrument VAPO SCAN AS-VT100RS (ASAHI BIOMED) according to the manufacturer's manual. The results are shown in FIGS. 15(a) and 15(b).

As apparent from FIG. 15(a), in the developing NC/Nga mouse as a control, the TEWL after 8 weeks was as high a value as about 60 g/h·m², whereas, in the developing NC/Nga mouse to which Compound 1 was orally administered, the TEWL after 8 weeks was as low a value as about 10 g/h·m². The TEWL of the non-developing NC/Nga mouse was from 5 to 10 g/h·m², and hence it was found that Compound 1 suppressed the amount of water transpiration to the same degree as the non-developing mouse. In addition, from FIG. 15(b), it was found that 4 weeks after the end of oral administration (12 weeks after the start of oral administration), the amount of water transpiration in the Compound 1-administered mouse was suppressed as compared to the control. From these results, it was found that Compound 1 was useful for alleviating the stratum corneum abnormality in the skin associated with atopic dermatitis.

### (iii) Appearance of developing NC/Nga mouse 12 weeks after start of oral administration

The appearances of the developing NC/Nga mice 12 weeks after the start of oral administration (the oral administration was ended 8 weeks after the start of oral administration) in (i) described above were shown in FIGS. 16(a) and 16(b).

As apparent from FIG. 16(a), in the control mouse, clinical manifestations at the parts around eyes, those of the head, and those of the ear auricles were extremely bad. In addition, in the control mouse, hair loss was also observed in the head. On the other hand, as apparent from FIG. 16(b), in the Compound 1-administered mouse, almost good clinical manifestations were kept even 4 weeks after the end of oral administration, though the recurrence of mild eczema in parts of ear auricles was partially observed.

### (Comparative Example 2) Technique described in Patent Literature 6 (JP 2004-175687 A)

Patent Literature 6 (JP 2004-175687 A) describes that tape stripping was applied to the skin of a hairless mouse to physically break the skin barrier, and thereafter, the barrier was restored by decreasing the calcium level in cells by applying a cAMP antagonist.

The FLG mRNA expression-promoting effect was confirmed for many compounds given as examples of the cAMP antagonist in the patent literature ((-)-quinpirole hydrochloride, (+)-PD128907, PD168077, 8-hydroxy-PIPAT, oxotremorine, (S)-3,4-DCPG, SNC80, SB205607, BRL-52537, SCH23390, CY208-243, RS39604, RS23597-190, pimozide, ICI-118,551, and betaxolol).

HaCaT cells were treated with each of the above-mentioned compounds at a concentration of from 1 to 10 µM under the culture conditions described in J. Investigative Dermatology (2011) 131: 1660-1667, and then total RNA was extracted to measure FLG mRNA by RT-PCR, but no FLG mRNA expression was observed. From this result, it was confirmed that the cAMP antagonist did not necessarily have the FLG production-promoting activity.

On the other hand, as described in each of Examples described above, a GPR142 agonist has an FLG production-promoting action while having an action as a cAMP antagonist. Accordingly, it was confirmed that the invention of the present application clearly differed from the invention described in Patent Literature 6.

### (Example 15) FLG monomer-producing effect in atopic dermatitis mouse model (NC/Nga)

Experiments were conducted with raising 6- to 10-week-old atopic dermatitis-developing NC/Nga mice with mites (Japan SLC, Inc.) under a normal environment. Namely, 500 µl of a solution obtained by dissolving Compound 1 at 1.5 mg/ml in a methyl cellulose solution (0.5 W/V% Methyl Cellulose 400 Solution, Wako, 133-14255) were orally administered to an NC/Nga mouse developing dermatitis majorly in the ear auricles and the head (0.75 mg of Compound 1 was administered to the mouse per time) every other day. As a control, 500 µl of the methyl cellulose solution was orally administered every other day. The experiments were conducted by using five mice in each of a Compound 1-administered group and a control group.

2 weeks after the beginning of administration, ears of each mouse were cut under anesthesia, the skin of the cut ear was peeled off by pinching cartilage, and the skin on the dermal side was soaked in PBS containing 20 mM EDTA at 37°C for 30 minutes. Next, the epidermis of the ear was peeled off and 50 ml of Lysis buffer (0.1 M Tris-HCl (pH 9.0), 6 M Urea, 1% 2-mercapto-ethanol, 1% SDS, 1 mM EDTA, and Protease inhibitor cocktail) were added to the epidermis. The epidermis was homogenized with an electrically powered pestle, and the homogenized cells were centrifuged at 15,000 rpm at 4°C for 20 minutes. The supernatant was collected to prepare a sample for measurement, and the sample was subjected to electrophoresis, and then to western blotting to confirm the expression of FLG monomer. 1 µg/ml of an anti-mouse FLG antibody (Anti-Mouse Filaggrin Antibody, Covance Inc.) was used as a primary antibody, Anti-Rabbit IgG-HRP (GE Healthcare) was used as a secondary antibody, and Amersham ECL Plus Western Blotting Detection Reagents (GE Healthcare) was used as a color developer.

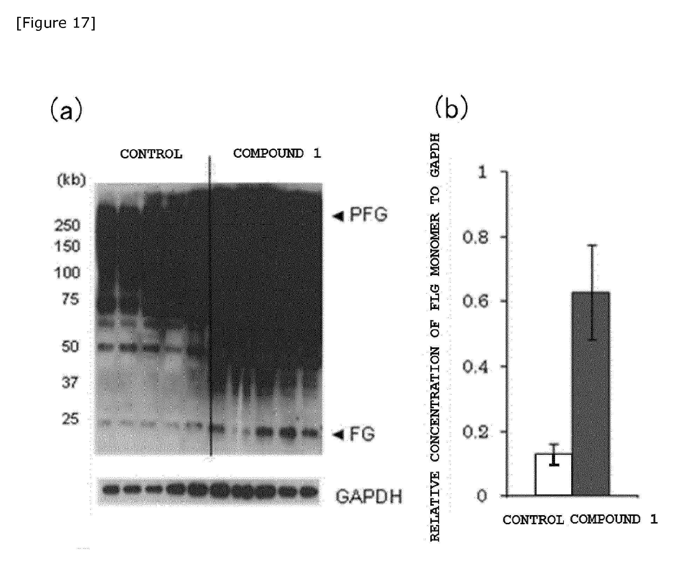

The result of the electrophoresis is shown in FIG. 17(a). PFG indicates the band of profilaggrin and FG indicates the band of FLG monomer. In addition, FIG. 17(b) graphically shows the relative concentration of FLG monomer to GAPDH (mean values of 5 samples). From these results, it was confirmed that the administration of Compound 1 clearly promoted the production of FLG monomer as compared to the control.

### Industrial Applicability

As described in detail above, it was confirmed that the GPR142 agonist, which was the active ingredient of the therapeutic agent according to the present invention, promoted FLG production in the skin, satisfactorily improves the water retention ability and barrier function of the stratum corneum, and acted as the active ingredient of the FLG production promoter. In addition, the GPR142 agonist can act as an active ingredient of a therapeutic agent for a skin disease associated with reduced FLG production. Further, one of the GPR142 agonists, that is, A) the N-quinolylbenzamide derivative (Compound 1), B) ivermectin (Compound 2), or C) the phenylalaninamide derivative (Compound 3) can be easily synthesized by chemical synthesis, and is useful as a therapeutic agent for a skin disease, in particular, atopic dermatitis associated with reduced FLG production. Moreover, by using GPR142 as a target, the therapeutic agent for a disease associated with reduced FLG production can be systematically screened.

## Claims

1. A therapeutic agent for a disease associated with reduced filaggrin (FLG) production, comprising a GPR142 agonist as an active ingredient.

2. A therapeutic agent for a disease according to claim 1, wherein the disease associated with reduced FLG production comprises a skin disease.

3. A therapeutic agent for a disease according to claim 2, wherein the skin disease comprises atopic dermatitis.

4. A therapeutic agent for a disease according to claim 2, wherein the skin disease comprises ichthyosis vulgaris.

5. A therapeutic agent for a disease according to any one of claims 1 to 4, wherein the GPR142 agonist comprises N-(4-amino-2-methyl-6-quinolyl)-2-[(4-ethylphenoxy)methyl]benzamide or a pharmaceutically acceptable salt thereof.

6. A therapeutic agent for a disease according to any one of claims 1 to 4, wherein the GPR142 agonist comprises a compound represented by the following general formula (I) or a pharmaceutically acceptable salt thereof: 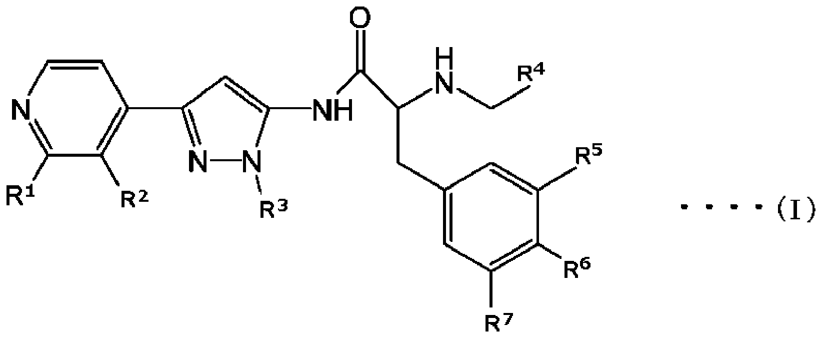 where R¹ and R² each represent a hydrogen atom, a halogen atom, or an alkyl group having 1 to 3 carbon atoms, R³ represents an alkyl group having 1 to 3 carbon atoms, R⁴ represents an alkoxycarbonyl group having 1 to 3 carbon atoms or a hydroxycarbonyl group, and R⁵, R⁶, and R⁷ each represent a hydrogen atom or a halogen atom.

7. A therapeutic agent for a disease according to claim 6, wherein, in the general formula (I) as described in claim 6, R¹ and R² each represent a hydrogen atom, R³ represents a methyl group, R⁴ represents an ethoxycarbonyl group or a hydroxycarbonyl group, and R⁵, R⁶, and R⁷ each represent a hydrogen atom.

8. A therapeutic agent for a disease according to any one of claims 1 to 4, wherein the GPR142 agonist comprises ivermectin.

9. A therapeutic agent for a disease associated with reduced FLG production for alleviating a symptom of the disease associated with reduced FLG production, the therapeutic agent for a disease associated with reduced FLG production comprising a GPR142 agonist as an active ingredient.

10. A therapeutic agent for a disease according to claim 9, wherein the disease associated with reduced FLG production comprises a skin disease.

11. A therapeutic agent for atopic dermatitis for alleviating a reduced water retention ability and/or barrier function of stratum corneum of skin associated with atopic dermatitis, the therapeutic agent for atopic dermatitis comprising a GPR142 agonist as an active ingredient.

12. An FLG production promoter, comprising a GPR142 agonist as an active ingredient.

13. A therapeutic method for a disease associated with reduced FLG production, comprising administering an effective amount of a medicament comprising a GPR142 agonist as an active ingredient to a patient presenting a symptom of a disease associated with reduced FLG production to alleviate the symptom of the patient.

14. A therapeutic method for atopic dermatitis, comprising administering an effective amount of a medicament comprising a GPR142 agonist as an active ingredient to a patient with atopic dermatitis with reduced FLG production to alleviate a stratum corneum abnormality of skin associated with the atopic dermatitis of the patient.

15. A screening method for a therapeutic agent for a disease associated with reduced FLG production, comprising evaluating whether a test substance is capable of promoting an activity or expression of GPR142 or not.

16. A screening method according to claim 15, wherein the screening method comprises the following steps (a) to (c):
(a) bringing a GPR142-expressing cell or a membrane fraction thereof into contact with a test substance;
(b) measuring an activity or expression of GPR142 in the cell or membrane fraction thereof after the contact with the test substance; and
(c) selecting a substance capable of promoting the activity or expression of GPR142 in the cell or membrane fraction thereof.

17. A screening method according to claim 15 or 16, wherein the disease associated with reduced FLG production is a skin disease.

18. A screening method according to claim 17, wherein the skin disease is atopic dermatitis.

19. A screening method according to any one of claims 15 to 18, wherein the GPR142-expressing cell comprises a cell obtained by transformation with an expression vector comprising a GPR142 gene.

20. A screening method according to claim 19, wherein the GPR142 gene comprises a human GPR142 gene having an amino acid sequence set forth in SEQ ID NO: 1 or a gene encoding a functional variant of human GPR142 that has an amino acid sequence with 90% or more identity to the amino acid sequence and retains an activity of GPR142.
